# EUROPÄISCHE PATENTANMELDUNG

(11) **EP 0 905 306 A1**
(43) Veröffentlichungstag der Anmeldung: **31.03.1999**
(21) Anmeldenummer: 98117193.7
(22) Anmeldetag: 10.09.1998
(51) Int. Cl.: D21C 5/00, D21C 9/10, C12S 3/00

(54) **Mehrkomponentensystem zum Verändern, Abbau oder Bleichen von Lignin, ligninhaltigen Materialien sowie Verfahren zu seiner Anwendung**

(30) Priorität: 26.09.1997 DE 19742671; 05.02.1998 DE 19804583
(71) Anmelder: Consortium für Elektrochemische Industrie GmbH, 81379 München (DE)
(72) Erfinder: Amann, Manfred, Dr., 85235 Odelzhausen (DE); Wohlschläger, Michael, 82223 Eichenau (DE); Freudenreich, Johannes, Dr., 80331 München (DE); Stohrer, Jürgen, Dr., 82049 Pullach (DE); Fritz-Langhals, Elke, Dr., 85521 Ottobrunn (DE)
(74) Vertreter: Potten, Holger

(57) **Zusammenfassung**

Die Erfindung betrifft ein Mehrkomponentensystem zum Verändern, Abbauen oder Bleichen von Lignin, ligninhaltigen Materialien oder ähnlichen Stoffen, enthaltend eine Oxidoreduktase und ein für die Oxidoreduktase geeignetes Oxidationsmittel und einen Mediator und mindestens ein enzymatisch wirksames Additiv, das dadurch gekennzeichnet ist, daß der Mediator die Oxidoreduktase und das enzymatisch wirksame Additiv nicht inaktiviert und das enzymatisch wirksame Additiv aus der Gruppe der Hydrolasen der Enzymklasse 3.2.1. ausgewählt ist.

## Beschreibung

Die vorliegende Erfindung betrifft ein Mehrkomponentensystem zum Verändern, Abbau oder gleichen von Lignin, ligninhaltigen Materialien sowie Verfahren zu seiner Anwendung.

Das Hauptziel aller Verfahren zur Herstellung von Zellstoff ist die Entfernung des Lignins aus dem pflanzlichen Ausgangsmaterial. Typischerweise wird dieses Ziel dadurch erreicht, daß geeignete oxidative oder reduktive chemische Prozeßschritte angewendet werden, welche das Lignin letztendlich in einer Weise modifizieren, in welcher es sich leicht aus dem Zellstoffprodukt extrahieren läßt. Traditionellerweise werden in den chemischen Prozeßschritten Chlor oder chlorhaltige Chemikalien, wie Hypochlorit oder Chlordioxid, verwendet.

In jüngerer Zeit werden anstelle dieser oder in Ergänzung zu diesen chlorhaltigen Verbindungen chlorfreie Chemikalien eingesetzt. Diese umfassen z.B. Sauerstoff, Wasserstoffperoxid, Peroxisäuren oder Ozon. Typischerweise wird der zu bearbeitende Zellstoff zwischen den verschiedenen chemischen Behandlungen einem Extraktionsschritt unterworfen, welcher Alkali, z.B. NaOH, enthält.

Als zusätzlicher chlorfreier Verfahrensschritt in den chemischen Verfahren zur Entfernung von Lignin sind enzymatische Verfahren bekannt, die die chemische Entfernung des Lignins in einer vielstufigen Delignifizier- und Bleichsequenz erleichtern sollen. Dieser Verfahrensschritt kann einstufig oder mehrstufig in sequentieller Kombination mit chemischen Verfahrensschritten durchgeführt werden (WO 91/11553). Als Vorteile dieses Verfahrensschritts werden die Verbesserung der erzielbaren Weißegrade oder die Reduktion des Chemikalienbedarfs, speziell der eingesetzten Chlormenge, genannt.

Aus EP 0 487 557 ist ferner bekannt, bei chemischen Delignifizierungsverfahren mit Hilfe von Sauerstoff zusätzlich einzelne Enzyme oder Mischungen von Enzymen aus verwandten Klassen einzusetzen. Dies sind z.B. Enzympräparationen, welche verschiedene Hemizellulasen enthalten.

Aus US 5,374,555 ist ein chemisches Verfahren mit zusätzlicher enzymatischer Zellstoffbehandlung unter Verwendung von Enzymen aus der Gruppe der Proteasen bekannt. Auch hier wird die Behandlung als ein separater Verfahrensschritt durchgeführt, welcher in sequentieller Kombination mit chemischen Verfahrensschritten zu Vorteilen führt.

WO 91/11552 beschreibt die Verwendung von oxidierenden und hydrolytischen Enzymen in einem Verfahren zur mechanischen Herstellung von Holzstoff. Zusätze wie z.B. Ascorbinsäure sollen dabei dazu dienen, das Redoxpotential auf Werte <200 mV einzustellen.

Neben den beschriebenen enzymatischen Verfahren, welche zusätzlich zu chemischen Verfahren zur Behandlung des Zellstoffs eingesetzt werden, sind auch enzymatische Verfahren zur Behandlung des Zellstoffs beschrieben.

Solche Verfahren nutzen phenol-oxydierende Enzyme. Beispiele für solche Enzyme sind Enzyme aus der Gruppe der Peroxidasen ( E.C. 1.11.1) und Oxidasen (E.C.1.10.3). Die Enzyme verwenden entweder Wasserstoffperoxid oder molekularen Sauerstoff als Elektronakzeptor. Der Einsatz dieser oxidativen Enzyme in der Bleiche ist beschrieben (z. B. Paice, M.G., 1995). Verwendung finden z.B. Mangan Peroxidase (MnP), Lignin Peroxidase (LiP) oder Laccase (E.C. 1.10.3.2). Während LiP und MnP als Cosubstrat typischerweise Wasserstoffperoxid benötigen, arbeitet Laccase mit Sauerstoff als Elektronenakzeptor.

Der erfolgreiche Einsatz von Peroxidasen in der Bleiche konnte noch nicht überzeugend geführt werden, da es bisher nicht möglich ist, das Problem der Peroxidase-Inaktivierung durch Wasserstoffperoxid bzw. die geeignete Dosierungstechnik für Wasserstoffperoxid in einem nicht kontinuierlichen technischen Verfahren zu lösen.

Während der alleinige Einsatz von Laccase das Lignin nicht in die gewünschte extrahierbare Form überführt, konnte durch Zugabe geeigneter Mediatorsubstanzen zu Laccase ein technisches Verfahren entwickelt werden, welches Zellstoff jeglicher Art erfolgreich delignifizieren kann. Ein solches enzymatische Delignifiziersystem, das aus den Komponenten Laccase, Mediator und Sauerstoff besteht, ist in WO 94/29510 beschrieben. Diese Anmeldung gibt zudem ausführlich den Stand der Technik wieder.

Weitere Mediatoren für enzymatischen Delignifizierungen unter Verwendung von Laccase sind in WO 97/06244 beschrieben.

WO 94/01538 beschreibt die Verwendung von Cellobiose-Oxidase in Kombination mit einer Endoglycanase, beispielsweise Xylanase und/oder Oxidoreduktase, beispielsweise Laccase. In dem Verfahren wird darüber hinaus noch ein Bleichverstärker zugegeben. Die angegebenen Bedingungen nennen unter anderem einen alkalischen pH-Wert. Die Wirksamkeit eines solchen Systems ist sehr gering, da bei dem genannten pH-Wert die beschriebene Laccase weitgehend inaktiv ist. Zudem wird Laccase in Gegenwart von Wasserstoffperoxid inaktiviert. Hinzu kommt, daß die als Bleichverstärker genannten Verbindungen keine Verstärkeraktivität zeigen. Dies ist aus WO 96/12846 ersichtlich, wo eine der Zellstoffbleiche analoge Anwendung, die Bleiche von gefärbten Textilfasern, beschrieben wird:
WO 96/12846 beschreibt im Beispiel 1 die Wirksamkeit unterschiedlicher Mediatoren. Wie in diesem Beispiel aus Tabelle 2 ersichtlich, zeigen die dort genannten exemplarischen Verbindungen keine Mediatoraktivität.

WO 94/29510 beschreibt die vorteilhafte Kombination eines enzymatischen Delignifiziersystems bestehend aus einer Laccase und einem aktiven Mediator. Dieses Verfahren ist das gegenwärtig einzige bekannte enzymatische Verfahren, welches zu einem effektiven Abbau von Lignin führt. Als Mediator wird dabei N-OH-Benzotriazol (HBT) verwendet. In dem beschriebenen Verfahren wird unter anderem auch der Zusatz weiterer enzymatischer Komponenten, wie z.B. Proteasen, erwähnt.

Wie von Oksanen et al. 1997 gezeigt, weist eine kombinierte Anwendung von Xylanase/Laccase/HBT nur eine sehr geringe Verbesserung gegenüber einer xylanasefreien Anwendung auf. Vorteile ergaben sich erst, nachdem die Xylanasebehandlung von der Laccase/HBT-Behandlung abgetrennt wurde und als eigener Verfahrensschritt durchgeführt wurde.

Es ist daher wünschenswert, ein Mehrkomponentensystem zum Verändern, Abbau oder Bleichen von Lignin und ligninhaltigen Materialien zur Verfügung zu haben, welches bessere Ergebnisse in der Delignifizierung erreicht als bekannte enzymatische Delignifizierungssysteme und nicht mit den Nachteilen von chemischen Delignifizierungssystemen behaftet ist.

Neben der schlechten Performance der einzelnen enzymatischen Verfahren zur Zellstoffdelignifizierung stellt die aufgrund der oben geschilderten Probleme notwendige Vereinzelung von enzymatischen Delignifizierstufen und das daraus resultierende schrittweise Vorgehen bei der Delignifizierung von Zellstoff einen beträchtlichen Nachteil dieser Verfahren dar. Durch die Notwendigkeit von Wasch- und Extraktionsschritten zwischen den unterschiedlichen Enzymbehandlungen lassen sich die Verfahren nicht wirtschaftlich in die bestehenden Delignifizier- und Bleichsequenzen integrieren.

Es ist daher ebenfalls wünschenswert, enzymatische Verfahren zur Behandlung von Lignin und ligninhaltigen Materialien bereitzustellen, die mehrere enzymatische Verfahrensschritte in einer einzigen Verfahrensstufe vereinigen.

Die vorliegende Erfindung betrifft ein Mehrkomponentensystem zum Verändern, Abbau oder Bleichen von Lignin, ligninhaltigen Materialien oder ähnlichen Stoffen, enthaltend eine Oxidoreduktase, ein für die Oxidoreduktase geeignetes Oxidationsmittel, einen Mediator und mindestens ein enzymatisch wirksames Additiv, dadurch gekennzeichnet, daß der Mediator die Oxidoreduktase und das enzymatisch wirksame Additiv nicht inaktiviert und das enzymatisch wirksame Additiv aus der Gruppe der Hydrolasen der Enzymlasse 3.2.1. ausgewählt ist.

Die Benennüng der Enzymklassen erfolgt in der vorliegenden Anmeldung gemäß Internationaler Enzym-Nomenklature, Committee of the International Union of Biochemistry and Molecular Biology (Enzyme Nomenclature, Academic Press, Inc., 1992, S. 24-154).

Im Sinne der Erfindung inaktiviert ein Mediator ein Enzym, wenn er einen Verlust von > 70 % der Enzymaktivität dieses Enzyms innerhalb einer Inkubationszeit von 30 min. in einem Testsystem bewirkt, welches in einem Gesamtvolumen von 50 ml bei 45°C 60 IU Oxidoreduktase, beispielsweise Laccase und 400 IU Hydrolase der Enzymklasse 3.2.1, beispielsweise Xylanase, Zellulase oder eine entsprechende Menge eines- anderen Enzyms, sowie 7,5 mmol/l Mediator enthält.

Das erfindungsgemäße Mehrkomponentensystem weist folgende Vorteile gegenüber dem Stand der Technik auf:
- Es besitzt eine hohe Selektivität für Lignin
- Es beeinträchtigt die Fasergualität nicht negativ
- Es ermöglicht in Kombination mit chemischen Delignifizierungsverfahren eine Chemikalieneinsparung in der Gesamtdelignifizierung und Bleiche
- Es stellt eine Verbesserung der bisherigen Einzelverfahren von Xylanase-Behandlung und Laccase-Mediator-System dar. Die Verbesserung ist größer als die Summe der Einzelverfahren.

Der Einsatz von Zellulase in Verfahren zur Herstellung von Zellstoff ist bisher unbekannt. Da Zellulasen bekanntermaßen Zellulose abbauen und damit nach bisheriger Meinung Zellstoff immer schädigen, ist ihr Einsatz im Verfahren zur Herstellung von Zellstoff für den Fachmann völlig unerwartet und überraschend.

Als Oxidoreduktasen können im erfindungsgemäßen Mehrkomponentensystem Oxidoreduktasen der Klassen 1.1.1 bis 1.97 gemäß Internationaler Enzym-Nomenklature, Committee of the International Union of Biochemistry and Molecular Biology (Enzyme Nomenclature, Academic Press, Inc., 1992, S. 24-154) eingesetzt werden.

Vorzugsweise werden Oxidoreduktasen der im Folgenden genannten Klassen eingesetzt:

Enzyme der Klasse 1.1, die alle Dehydrogenasen, die auf primäre, sekundäre Alkohole und Semiacetale wirken, umfassen, und die als Akzeptoren NAD⁺ oder NADP⁺ (Subklasse 1.1.1), Cytochrome (1.1.2), Sauerstoff (O₂) (1.1.3), Disulfide (1.1.4), Chinone (1.1.5) oder andere Akzeptoren haben (1.1.99).

Aus dieser Klasse sind besonders bevorzugt die Enzyme der Klasse 1.1.5 mit Chinonen als Akzeptoren und die Enzyme der Klasse 1.1.3 mit Sauerstoff als Akzeptor.

Insbesondere bevorzugt in dieser Klasse ist Cellobiose: quinone-1-oxidoreduktase (1.1.5.1).

Weiterhin bevorzugt sind Enzyme der Klasse 1.2. Diese Enzymklasse umfaßt solche Enzyme, die Aldehyde zu den korrespondierenden Säuren oder Oxo-Gruppen oxidieren. Die Akzeptoren können NAD⁺, NADP⁺ (1.2.1), Cytochrome (1.2.2), Sauerstoff (1.2.3); Sulfide (1.2.4), Eisen-Schwefel-Proteine (1.2.5) oder andere Akzeptoren (1.2.99) sein.

Besonders bevorzugt sind hier die Enzyme der Gruppe (1.2.3) mit Sauerstoff als Akzeptor.

Weiterhin bevorzugt sind Enzyme der Klasse 1.3.

In dieser Klasse sind Enzyme zusammengefaßt, die auf CH-CH-Gruppen des Donors wirken.

Die entsprechenden Akzeptoren sind NAD⁺, NADP⁺ (1.3.1), Cytochrome (1.3.2), Sauerstoff (1.3.3), Chinone oder verwandte Verbindungen (1.3.5), Eisen-Schwefel-Proteine (1.3.7) oder andere Akzeptoren (1.3.99).

Besonders bevorzugt ist die Bilirubinoxidase (1.3.3.5).

Hier sind ebenfalls die Enzyme der Klasse (1.3.3) mit Sauerstoff als Akzeptor und (1.3.5) mit Chinonen etc. als Akzeptor besonders bevorzugt.

Weiterhin bevorzugt sind Enzyme der Klasse 1.4, die auf CH-NH₂-Gruppen des Donors wirken.

Die entsprechenden Akzeptoren sind NAD⁺, NADP⁺ (1.4.1), Cytochrome (1.4.2), Sauerstoff (1.4.3), Disulfide (1.4.4), Eisen-Schwefel-Proteine (1.4.7) oder andere Akzeptoren (1.4.99).

Besonders bevorzugt sind auch hier Enzyme der Klasse 1.4.3 mit Sauerstoff als Akzeptor.

Weiterhin bevorzugt sind Enzyme der Klasse 1.5, die auf CH-NH-Gruppen des Donors wirken. Die entsprechenden Akzeptoren sind NAD⁺, NADP⁺ (1.5.1), Sauerstoff (1.5.3), Disulfide (1.5.4), Chinone (1.5.5) oder andere Akzeptoren (1.5.99).

Auch hier sind Enzyme mit Sauerstoff (O₂) (1.5.3) und mit Chinonen (1.5.5) als Akzeptoren besonders bevorzugt.

Weiterhin bevorzugt sind Enzyme der Klasse 1.6, die auf NADH oder NADPH wirken.

Die Akzeptoren sind hier NADP⁺ (1.6.1), Hämproteine (1.6.2), Disulfide (1.6.4), Chinone (1.6.5), NO₂-Gruppen (1.6.6) und ein Flavin (1.6.8) oder einige andere Akzeptoren (1.6.99).

Besonders bevorzugt sind hier Enzyme der Klasse 1.6.5 mit Chinonen als Akzeptoren.

Weiterhin bevorzugt sind Enzyme der Klasse 1.7, die auf andere NO₂-Verbindungen als Donatoren wirken und als Akzeptoren Cytochrome (1.7.2), Sauerstoff (O₂) (1.7.3), Eisen-Schwefel-Proteine (1.7.7) oder andere (1.7.99) haben.

Hier ist besonders bevorzugt die Klasse 1.7.3 mit Sauerstoff als Akzeptor.

Weiterhin bevorzugt sind Enzyme der Klasse 1.8, die auf Schwefelgruppen als Donatoren wirken und als Akzeptoren NAD⁺, NADP⁺ (1.8.1), Cytochrome (1.8.2), Sauerstoff (O₂) (1.8.3), Disulfide (1.8.4), Chinone (1.8.5), Eisen-Schwefel-Proteine (1.8.7) oder andere (1.8.99) haben.

Besonders bevorzugt ist die Klasse 1.8.3 mit Sauerstoff (O₂) und (1.8.5) mit Chinonen als Akzeptoren.

Weiterhin bevorzugt sind Enzyme der Klasse 1.9, die auf Hämgruppen als Donatoren wirken und als Akzeptoren Sauerstoff (O₂) (1.9.3), NO₂-Verbindungen (1.9.6) und andere (1.9.99) haben.

Besonders bevorzugt ist hier die Gruppe 1.9.3 mit Sauerstoff (O₂) als Akzeptor (Cytochromoxidasen).

Weiterhin bevorzugt sind Enzyme der Klasse 1.12, die auf Wasserstoff als Donor wirken.

Die Akzeptoren sind NAD⁺ oder NADP⁺ (1.12.1) oder andere (1.12.99).

Des weiteren bevorzugt sind Enzyme der Klasse 1.13 und 1.14 (Oxigenasen).

Weiterhin sind bevorzugte Enzyme die der Klasse 1.15 , die auf Superoxid-Radikale als Akzeptoren wirken.

Besonders bevorzugt ist hier die Superoxid-Dismutase (1.15.1.1).

Weiterhin sind bevorzugt Enzyme der Klasse 1.16. Als Akzeptoren wirken NAD⁺ oder NADP⁺ (1.16.1) oder Sauerstoff (O₂) (1.16.3).

Besonders bevorzugt sind hier Enzyme der Klasse 1.16.3.1 (Ferroxidase, z.B. Ceruloplasmin).

Weiterhin bevorzugte Enzyme sind diejenigen, die der Gruppe 1.17 (Wirkung auf CH₂-Gruppen, die zu -CHOH- oxidiert werden), 1.18 (Wirkung auf reduziertes Ferredoxin als Donor), 1.19 (Wirkung auf reduziertes Flavodoxin als Donor) und 1.97 (andere Oxidoreduktasen) angehören.

Weiterhin besonders bevorzugt sind die Enzyme der Gruppe 1.11., die auf ein Peroxid als Akzeptor wirken. Diese einzige Subklasse (1.11.1) enthält die Peroxidasen.

Besonders bevorzugt sind hier die Cytochrom-C-Peroxidasen (1.11.1.5), Catalase (1.11.1.6), die Peroxydase (1.11.1.6), die Iodid-Peroxidase (1.11.1.8), die Glutathione-Peroxidase (1.11.1.9), die Chlorid-Peroxidase (1.11.1.10), die L-Ascorbat-Peroxidase (1.11.1.11), die Phospholipid-Hydroperoxid- Glutathione-Peroxidase (1.11.1.12), die Mangan-Peroxidase (1.11.1.13), die Diarylpropan-Peroxidase (Ligninase, Lignin-Peroxidase) (1.11.1.14).

Ganz besonders bevorzugt sind Enzyme der Klasse 1.10, die auf Biphenole und verwandten Verbindungen wirken. Sie katalysieren die Oxidation von Biphenolen und Ascorbaten. Als Akzeptoren fungieren NAD⁺, NADP⁺ (1.10.1), Cytochrome (1.10.2), Sauerstoff (1.10.3) oder andere (1.10.99).

Von diesen wiederum sind Enzyme der Klasse 1.10.3 mit Sauerstoff (O₂) als Akzeptor besonders bevorzugt.

Von den Enzymen dieser Klasse sind die Enzyme Catechol Oxidase (Tyrosinase) (1.10.3.1), L-Ascorbate Oxidase (1.10.3.3), o-Aminophenol Oxidase (1.10.3.4) und Laccase (Benzoldiol: Oxigen Oxidoreduktase) (1.10.3.2) bevorzugt, wobei die Laccasen (Benzoldiol: Oxigen Oxidoreduktase) (1.10.3.2) insbesondere bevorzugt sind.

Die genannten Oxidoreduktasen sind käuflich erhältlich oder lassen sich nach Standardverfahren gewinnen. Als Organismen zur Produktion der Enzyme kommen beispielsweise Pflanzen, tierische Zellen, Bakterien und Pilze in Betracht. Grundsätzlich können sowohl natürlich vorkommende als auch gentechnisch veränderte Organismen Enzymproduzenten sein. Ebenso sind Teile von einzelligen oder mehrzelligen Organismen als Enzymproduzenten denkbar, vor allem Zellkulturen.

Für die insbesondere bevorzugten Oxidoreduktasen, wie die aus der Gruppe 1.11.1, vor allem aber 1.10.3, und insbesondere zur Produktion von Laccasen werden beispielsweise Weißfäulepilze, wie Pleurotus, Phlebia und Trametes, verwendet.

Das erfindungsgemäße Mehrkomponentensystem umfaßt mindestens ein Oxidationsmittel. Als Oxidationsmittel können beispielsweise Luft, Sauerstoff, Ozon, H₂O₂, organische Peroxide, Persäuren wie die Peressigsaure, Perameisensäure, Perschwefelsäure, Persalpetersäure, Metachlorperoxibenzosäure, Perchlorsäure, Perborate, Peracetate, Persulfate, Peroxide oder Sauerstoffspezies und deren Radikale wie OH, OOH, Singulettsauerstoff, Superoxid (O₂⁻), Ozonid, Dioxygenyl-Kation (O₂⁺), Dioxirane, Dioxetane oder Fremy Radikale eingesetzt werden.

Vorzugsweise werden solche Oxidationsmittel eingesetzt, die entweder durch die entsprechenden Oxidoreduktasen generiert werden können, z.B. Dioxirane aus Laccasen plus Carbonylen, oder die chemisch den Mediator regenerieren oder diesen direkt umsetzen können.

Beispielsweise benötigen Peroxidasen als Cosubstrat Wasserstoffperoxid. Diese Verbindung kann entweder direkt in das System gegeben werden oder aus einer Vorläuferverbindung freigesetzt werden. Schließlich kann Wasserstoffperoxid auch in situ durch eine Hilfsreaktion gebildet werden, z.B. durch den enzymatischen Umsatz von Glucose mit Glucoseoxidase (E.C. 1.1.3.4 ).

Beispielsweise verwenden Oxidasen als Elektronenakzeptor Sauerstoff. Sauerstoff ist üblicherweise in ausreichender Menge in wässrigen Lösungen gelost. Sauerstoff kann aber auch durch geeignete Maßnahmen, wie Rühren, Verwendung von Sauerstoffgas oder das Anlegen von Druck, in die Reaktionslösung eingebracht werden. Dies ist besonders dann nötig, wenn das Verfahren bei erhöhter Temperatur betrieben werden soll, da die Löslichkeit von Sauerstoff in wässrigen Lösungen mit Zunahme der Temperatur abnimmt.

Das erfindungsgemäße Mehrkomponentensystem umfaßt mindestens einen Mediator, der die Oxidoreduktase und das enzymatisch wirksame Additiv nicht inaktiviert.

Als Mediator wird vorzugsweise mindestens eine Verbindung aus der Gruppe der aliphatischen, cycloaliphatischen, heterocyclischen oder aromatischen Verbindungen ausgewählt, die mindestens eine N-Hxdroxy, Oxim-, Nitroso-, N-Oxyl- oder N-Oxi-Funktion enthält, wobei substituierte oder nichtsubstituierte 1-Hydroxy-1-benzotriazole, 3H-Benzotriazol-1-oxide und 2H-Benzotriazol-1-oxide ausgenommen sind.

Beispiele für solche Verbindungen sind die im Folgenden genannten Verbindungen der Formel I, II, oder III, wobei die Verbindungen der Formeln II und III bevorzugt sind.
Verbindungen der allgemeinen Formel I sind: 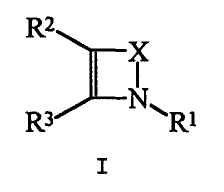 wobei X für eine der folgenden Gruppen steht:
(-N=CR⁴-)ₚ, (-CR⁴=N-)ₚ, (-CR⁵=CR⁶)ₚ
und p gleich 1 oder 2 ist,
wobei die Reste R¹ bis R⁶ gleich oder verschieden sein können und unabhängig voneinander eine der folgenden Gruppen darstellen können: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-alkyl, Phenyl, Sulfono, Ester und Salze davon, Sulfamoyl, Carbamoyl, Phospho, Phosphono, Phosphonooxy und deren Salze und Ester, wobei die Amino-, Carbamoyl- und Sulfamoyl-Gruppen der Reste R¹ bis R⁶ weiterhin unsubstitiert oder ein- oder zweifach mit Hydroxy, C₁-C₃-Alkyl, C₁-C₃-Alkoxy substituiert sein können,
und wobei die Reste R² und R³ eine gemeinsame Gruppe -A- bilden können und -A- dabei eine der folgenden Gruppen repräsentiert: (-CR⁷=CR⁸-CR⁹=CR¹⁰-) oder (-CR¹⁰=CR⁹-CR⁸=CR⁷-).

Die Reste R⁷ bis R¹⁰ können gleich oder ungleich sein und unabhängig voneinander eine der folgenden Gruppen darstellen: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-alkyl, Phenyl, Sulfono, Ester und Salze davon, Sulfamoyl, Carbamoyl, Phospho, Phosphono, Phosphonooxy und deren Salze und Ester, wobei die Amino-, Carbamoyl- und Sulfamoyl-Gruppen der Reste R⁷ bis R¹⁰ weiterhin unsubstituiert oder ein- oder zweifach mit Hydroxy, C₁-C₃-Alkyl, C₁-C₃-Alkoxy substituiert sein können und wobei die C₁-C₁₂-Alkyl-, C₁-C₆-Alkyloxy-, Carbonyl-C₁-C₆-alkyl-, Phenyl-, Aryl-Gruppen der Reste R⁷ bis R¹⁰ unsubstituiert oder weiterhin ein- oder mehrfach mit dem Rest R¹¹ substituiert sein können und wobei der Rest R¹¹ eine der folgenden Gruppen darstellen kann: Wasserstoff, Halogen, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl, sowie deren Ester und Salze, wobei die Carbamoyl-, Sulfamoyl-, Amino-Gruppen des Restes R¹¹ unsubstituiert oder weiterhin ein- oder zweifach mit dem Rest R¹² substituiert sein können und wobei der Rest R¹² eine der folgenden Gruppen darstellen kann: Wasserstoff, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl.

Verbindungen der allgemeinen Formel II sind: 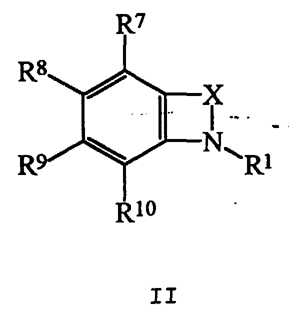 wobei X für eine der folgenden Gruppen steht:
(-N=CR⁴-)ₚ, (-CR⁴=N-)ₚ, (-CR⁵=CR⁶)ₚ
und p gleich 1 oder 2 ist.

Die Reste R¹ und R⁴ bis R¹⁰ können gleich oder ungleich sein und unabhängig voneinander eine der folgenden Gruppen darstellen: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-alkyl, Phenyl, Aryl, Sulfono, Ester und Salze davon, Sulfamoyl, Carbamoyl, Phospho, Phosphono, Phosphonooxy und deren Salze und Ester, wobei die Amino-, Carbamoyl- und Sulfamoyl-Gruppen der Reste R¹ und R⁴ bis R¹⁰ weiterhin unsubstituiert oder ein- oder zweifach mit Hydroxy, C₁-C₃-Alkyl, C₁-C₃-Alkoxy substituiert sein können
und wobei die C₁-C₁₂-Alkyl-, C₁-C₆-Alkyloxy-, Carbonyl-C₁-C₆-alkyl-, Phenyl-, Aryl-, Aryl-C₁-C₆-alkyl-Gruppen der Reste R¹ und R⁴ bis R¹⁰ unsubstituiert oder weiterhin ein- oder mehrfach mit dem Rest R¹² substituiert sein können und wobei der Rest R¹² eine der folgenden Gruppen darstellen kann: Wasserstoff, Halogen, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl, Sulfono, Sulfeno, Sulfino und deren Ester und Salze
und wobei die Carbamoyl-, Sulfamoyl-, Amino-Gruppen des Restes R¹² unsubstituiert oder weiterhin ein- oder zweifach mit dem Rest R¹³ substituiert sein können und wobei der Rest R¹³ eine der folgenden Gruppen darstellen kann: Wasserstoff, Hydroxy, Formyl, Carboxy sowie deren Salze und Ester, Amino, Nitro, C₁-C₁₂-Alkyl, C₁-C₆-Alkyloxy, Carbonyl-C₁-C₆-alkyl, Phenyl, Aryl.

Beispiele für die genannten Verbindungen sind:

### 1-Hydroxy-benzimdazole

1-Hydroxybenzimidazol-2-carbonsäure
1-Hydroxybenzimidazol
2-Methyl-1-hydroxybenzimidazol
2-Phenyl-1-hydroxybenzimidazol

### 1-Hydroxyindole

2-Phenyl-1-hydroxyindol

Substanzen der allgemeinen Formel III sind: 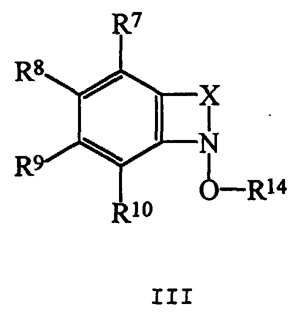 wobei X für eine der folgenden Gruppen steht:
(-N=CR⁴-)ₘ, (-CR⁴=N-)ₘ, (-CR⁵=CR⁶-)ₘ
und m gleich 1 oder 2 ist.

Für die Reste R⁷ bis R¹⁰ und R⁴ bis R⁶ gilt das oben gesagte.

R¹⁴ kann sein: Wasserstoff, C₁-C₁₀-Alkyl, C₁-C₁₀-Alkylcarbonyl, deren C₁-C₁₀-Alkyl und C₁-C₁₀-Alkylcarbonyl unsubstituiert oder mit einem Rest R¹⁵ ein- oder mehrfach substituiert sein können, wobei R¹⁵ eine der folgenden Gruppen darstellen kann: Wasserstoff, Halogen, Hydroxy, Formyl, Carboxy sowie Salze und Ester davon, Amino, Nitro, C₁-C₁₂-Alkyl, C₁-C₆-Alkyloxy, Carbonyl-C₁-C₆-Alkyl, Phenyl, Sulfono, deren Ester und Salze, Sulfamoyl, Carbamoyl, Phospho, Phosphono, Phosphonooxy und deren Salze und Ester,
wobei die Amino-, Carbamoyl- und Sulfamoyl-Gruppen des Restes R¹⁵ weiterhin unsubstituiert oder ein- oder zweifach mit Hydroxy, C₁-C₃-Alkyl, C₁-C₃-Alkoxy substituiert sein können.

Der Mediator kann vorzugsweise ferner ausgewählt sein aus der Gruppe cyclischer N-Hydroxyverbindungen mit mindestens einem ggf. substituierten fünf- oder sechsgliedrigen Ring, enthaltend die in Formel IV genannte Struktur 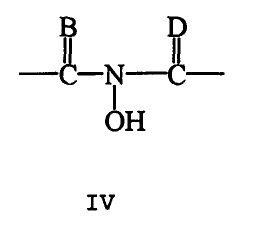 sowie deren Salze, Ether oder Ester, wobei
B und D gleich oder verschieden sind und O, S oder NR¹⁶ bedeuten, wobei
R¹⁶ Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeutet, wobei Carbamoyl-, Sulfamoyl-, Amino- und Phenylreste unsubstituiert oder ein- oder mehrfach mit einem Rest R¹⁷ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R¹⁷ ein- oder mehrfach substituiert sein können wobei R¹⁷ gleich oder verschieden ist und Hydroxy-, Formyl-, Carboxyrest, Ester oder Salz des Carboxyrests, Carbamoyl-, Sulfono-, Ester oder Salz des Sulfonorests, Sulfamoyl-, Nitro-, Amino-, Phenyl-, C₁-C₅-Alkyl-, C₁-C₅-Alkoxyrest bedeutet.

Vorzugsweise ist der Mediator ausgewählt aus der Gruppe der Verbindungen der allgemeinen Formel V, VI, VII oder VIII, 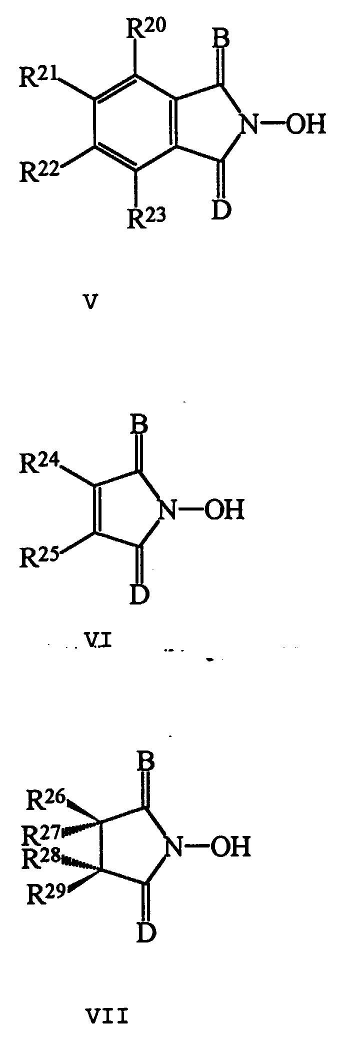 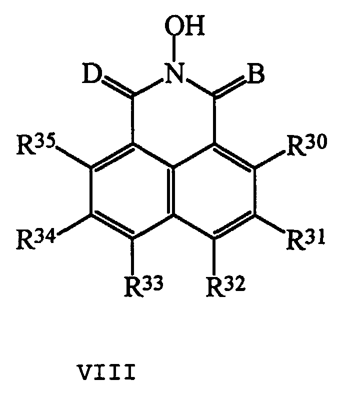 wobei B, D die bereits genannten Bedeutungen haben und
die Reste R¹⁸-R³³ gleich oder verschieden sind und Halogenrest, Carboxyrest, Salz oder Ester eines Carboxyrests sind oder die für R¹⁶ genannten Bedeutungen haben,
wobei R²⁴ und R²⁵ bzw. R²⁶ und R²⁷ nicht gleichzeitig Hydroxy- oder Aminorest bedeuten dürfen und
ggf. je zwei der Substituenten R¹⁸-R²¹, R²²-R²³, R²⁴-R²⁷, R²⁸-R³³ zu einem Ring -E- verknüpft sein können, wobei -E- eine der folgenden Bedeutungen hat:
(-CH=CH)-ₙ mit n = 1 bis 3, -CH=CH-CH=N- oder 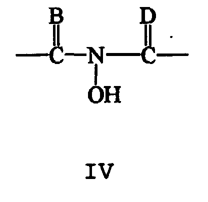 und wobei ggf. die Reste R²⁴-R²⁷ auch untereinander durch ein oder zwei Brückenelemente -F- verbunden sein können, wobei -F- gleich oder verschieden ist und eine der folgende Bedeutungen hat: -O-, -S, -CH₂-, -CR³⁴=CR³⁵- ;
wobei R³⁴ und R³⁵ gleich oder verschieden sind und die Bedeutung von R¹⁸ haben.

Als Mediatoren besonders bevorzugt sind Verbindungen der allgemeinen Formeln V, VI, VII oder VIII, bei denen B und D O oder S bedeuten.

Beispiele für solche Verbindungen sind N-Hydroxy-phthalimid sowie ggf. substituierte N-Hydroxy-phthalimid-Derivate, N-Hydroxymaleimid sowie ggf. substituierte N-Hydroxymaleimid-Derivate, N-Hydroxy-Naphthalsäureimid sowie ggf. substituierte N-Hydroxy-Naphthalsäureimid-Derivate, N-Hydroxysuccinimid und ggf.substituierte N-Hydroxysuccinimid-Derivate, vorzugsweise solche, bei denen die Reste R²⁴-R²⁷ polycyclisch verbunden sind.

Als Mediator insbesondere bevorzugt sind N-Hydroxyphthalimid, 4-Amino-N-Hydroxyphthalimid und 3-Amino-N-Hydroxyphthalimid.

Als Mediator geeignete Verbindungen der Formel V sind beispielsweise:
N-Hydroxyphthalimid,
4-Amino-N-Hydroxyphthalimid,
3-Amino-N-Hydroxyphthalimid,
N-Hydroxy-benzol-1,2,4-tricarbonsäureimid,
N,N'-Dihydroxy-pyromellitsäurediimid,
N,N'-Dihydroxy-benzophenon-3,3,4,4'-tetracarbonsäurediimid.

Als Mediator geeignete Verbindungen der Formel VI sind beispielsweise:
N-Hydroxymaleimid,
Pyridin-2,3-dicarbonsäure-N-hydroxyimid.

Als Mediator geeignete Verbindungen der Formel VII sind beispielsweise:
N-Hydroxysuccinimid,
N-Hydroxyweinsäureimid,
N-Hydroxy-5-norbornen-2,3-dicarbonsäureimid,
exo-N-Hydroxy-7-oxabicyclo[2.2.1]-hept-5-en-2,3-dicarboximid,
N-Hydroxy-cis-cyclohexan-1,2-dicarboximid,
N-Hydroxy-cis-4-cyclohexen-1,2-dicarbonsäureimid.

Als Mediator geeignete Verbindung der Formel VIII ist beispielsweise:
N-Hydroxynapthalsäureimid-Natrium-Salz.

Als Mediator geeignete Verbindung mit einem sechsgliedrigen Ring enthaltend die in Formel IV genannte Struktur ist beispielsweise:
N-Hydroxyglutarimid.

Die beispielhaft genannten Verbindungen eignen sich auch in Form ihrer Salze oder Ester als Mediator.

Als Mediator ebenfalls geeignet sind Verbindungen, ausgewählt aus der Gruppe der N-Aryl-N-Hydroxy-Amide.

Von diesen werden bevorzugt als Mediatoren eingesetzt Verbindungen der allgemeinen Formel IX, X oder XI 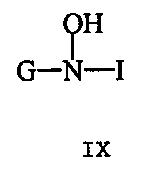 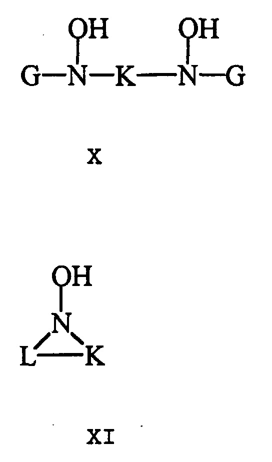 sowie deren Salze, Ether oder Ester, wobei
G einbindiger homo- oder heteroaromatischer ein- oder zweikerniger Rest und
L zweibindiger homo- oder heteroaromatischer ein- oder zweikerniger Rest bedeutet und
   wobei diese Aromaten durch einen oder mehrere, gleiche oder verschiedene Reste R³⁶, ausgewählt aus der Gruppe Halogen-, Hydroxy-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests substituiert sein können und
   wobei Carbamoyl-, Sulfamoyl-, Amino- und Phenylreste unsubstituiert oder ein- oder mehrfach mit einem Rest R³⁷ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R³⁷ ein- oder mehrfach substituiert sein können, wobei
R³⁷ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxy-, C₁ - C₅-Alkylcarbonylrest bedeutet und
je zwei Reste R³⁶ oder R³⁷ paarweise über eine Brücke [-CR³⁸R³⁹-]ₘ mit m gleich 0,1,2, 3 oder 4 verknüpft sein können und
R³⁸ und R³⁹ gleich oder verschieden sind und Carboxyrest, Ester oder Salz des Carboxyrests, Phenyl-, C₁-C₅-Alkyl-, C₁ -C₅-Alkoxy-, C₁ - C₅-Alkylcarbonylrest bedeuten und eine oder mehrere nicht benachbarte Gruppen [-CR³⁸R³⁹-] durch Sauerstoff, Schwefel oder einen ggf. mit C₁ bis C₅ Alkylrest substituierten Iminorest und zwei benachbarte Gruppen [-CR³⁸R³⁹-] durch eine Gruppe [-CR³⁸=CR³⁹-] ersetzt sein können und
I in amidischer Form vorliegender einbindiger Säurerest von Säuren, ausgewählt aus der Gruppe Carbonsäure mit bis zu 20 C-Atomen, Kohlensäure, Halbester der Kohlensäure oder der Carbaminsäure, Sulfonsäure, Phosphonsäure, Phosphorsäure, Monoester der Phosphorsäure, Diester der Phosphorsäure bedeutet und
K in amidischer Form vorliegender zweibindiger Säurerest von Säuren, ausgewählt aus der Gruppe Mono- und Dicarbonsäuren mit bis zu 20 C-Atomen, Kohlensäure, Sulfonsäure, Phosphonsäure, Phosphorsäure, Monoester der Phosphorsäure bedeutet.

Als Mediatoren besonders bevorzugt sind Verbindungen der allgemeinen Formel XII, XIII, XIV, XV oder XVI: 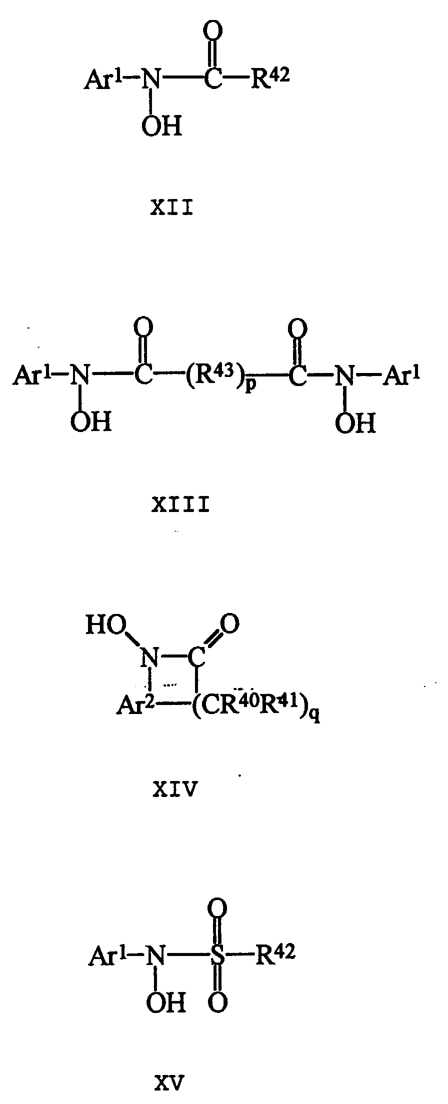 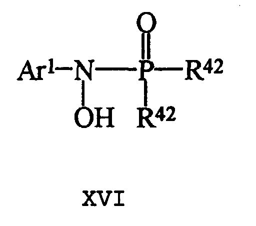 sowie deren Salze, Ether oder Ester, wobei
Ar¹ einbindiger homo- oder heteroaromatischer einkerniger Arylrest und
Ar² zweibindiger homo- oder heteroaromatischer einkerniger Arylrest bedeutet,
die durch eine oder mehrere, gleiche oder verschiedene Reste R⁴², ausgewählt aus der Gruppe Hydroxy-, Cyano-, Carboxyrest, Ester oder Salz des Carboxyrests, Sulfonorest, Ester oder Salz des Sulfonorests, Nitro-, Nitroso-, Amino-, C₁-C₁₂-Alkyl-, C₁-C₅-Alkoxy-, C₁-C₁₀-Carbonyl-, Carbonyl-C₁-C₆-alkylrest substituiert sein können,
   wobei Aminoreste unsubstituiert oder ein- oder mehrfach mit einem Rest R⁴³ substituiert sein können und die C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁴³ ein- oder mehrfach substituiert sein können,
   wobei R⁴³ gleich oder verschieden ist und Hydroxy-, Carboxyrest, Ester oder Salz des Carboxyrests, Sulfono-, Nitro-, Amino-, C₁-C₅-Alkyl-, C₁ - C₅-Alkoxy-, C₁ - C₅-Alkylcarbonylrest bedeutet und
je zwei Reste R⁴² paarweise über eine Brücke [-CR³⁸R³⁹-]ₘ mit m gleich 0, 1, 2, 3 oder 4 verknüpft sein können und
R³⁸ und R³⁹ die bereits genannten Bedeutungen haben und eine oder mehrere nicht benachbarte Gruppen [-CR³⁸R³⁹-] durch Sauerstoff, Schwefel oder einen ggf. mit einem C₁ bis C₅ Alkylrest substituierten Iminorest und zwei benachbarte Gruppen [-CR³⁸R³⁹-] durch eine Gruppe [-CR³⁸=CR³⁹-] ersetzt sein können,
R⁴⁰ gleiche oder verschiedene einbindige Reste ausgewählt aus der Gruppe Wasserstoff-, Phenyl-, Aryl-C₁-C₅-alkyl-, C₁-C₁₂-Alkyl-, C₁-C₅-Alkoxy-, C₁-C₁₀-Carbonylrest bedeutet, wobei Phenylreste unsubstituiert oder ein- oder mehrfach mit einem Rest R⁴⁴ substituiert sein können und die Aryl-C₁-C₅-alkyl-, C₁-C₁₂-Alkyl-, C₁-C₅-Alkoxy-, C₁-C₁₀-Carbonyl-Reste gesättigt oder ungesättigt, verzweigt oder unverzweigt sein können und mit einem Rest R⁴⁴ ein- oder mehrfach substituiert sein können, wobei
R⁴⁴ gleich-oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxyrest bedeutet und
R⁴¹ zweibindige Reste, ausgewählt aus der Gruppe ortho-, meta-, para-Phenylen-, Aryl-C₁-C₅-alkyl-, C₁-C₁₂-Alkylen-, C₁-C₅-Alkylendioxyrest bedeutet, wobei Phenylenreste unsubstituiert oder ein- oder mehrfach mit einem Rest R⁴⁴ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁴⁴ ein- oder mehrfach substituiert sein können, wobei
p 0 oder 1 bedeutet und
q eine ganze Zahl von 1 bis 3 bedeutet.

Vorzugsweise bedeutet Ar¹ Phenylrest und
Ar² ortho-Phenylenrest, wobei Ar¹ durch bis zu fünf und Ar² durch bis zu vier gleiche oder verschiedene Reste ausgewählt aus der Gruppe C₁-C₃-Alkyl-, C₁-C₃-Alkylcarbonyl-, Carboxyrest, Ester oder Salz des Carboxyrests, Sulfonorest, Ester oder Salz des Sulfonorests, Hydroxy-, Cyano-, Nitro-, Nitroso- und Aminorest substituiert sein können, wobei Aminoreste mit zwei verschiedenen Resten, ausgewählt aus der Gruppe Hydroxy und C₁-C₃-Alkylcarbonyl substituiert sein können.

Vorzugsweise bedeutet R⁴⁰ einbindiger Rest ausgewählt aus der Gruppe Wasserstoff-, Phenyl-, C₁-C₁₂-Alkyl-, C₁-C₅-Alkoxyrest, wobei die C₁-C₁₂-Alkylreste und C₁-C₅-Alkoxyreste gesättigt oder ungesättigt, verzweigt oder unverzweigt sein können.

Vorzugsweise bedeutet R⁴¹ zweibindige Reste, ausgewählt aus der Gruppe ortho- oder para-Phenylen-, C₁-C₁₂-Alkylen-, C₁-C₅-Alkylendioxyrest, wobei die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁴⁴ ein- oder mehrfach substituiert sein können.

Vorzugsweise bedeutet R⁴⁴ Carboxyrest, Ester oder Salz des Carboxyrests, Carbamoyl-, Phenyl-, C₁ - C₃-Alkoxyrest.

Beispiele für Verbindungen, die als Mediatoren eingesetzt werden können, sind N-Hydroxyacetanilid, N-Hydroxypivaloylanilid, N-Hydroxyacrylanilid, N-Hydroxybenzoylanilid, N-Hydroxy-methylsulfonylanilid, N-Hydroxy-N-phenyl-methylcarbamat, N-Hydroxy-3-oxo-butyrylanilid, N-Hydroxy-4-cyanoacetanilid, N-Hydroxy-4-methoxyacetanilid, N-Hydroxyphenacetin, N-Hydroxy-2,3-dimethylacetanilid, N-Hydroxy-2-methylacetanilid, N-Hydroxy-4-methylacetanilid, 1-Hydroxy-3,4-dihydrochinolin-(1H)-2-on, N,N'-Dihydroxy-N,N'-diacetyl-1,3-phenylendiamin, N,N'-Dihydroxybernsteinsäuredianilid, N,N'-Dihydroxy-maleinsäuredianilid, N,N'-Dihydroxy-oxalsäuredianilid, N,N'-Dihydroxy-phosphorsäuredianilid, N-Acetoxyacetanilid, N-Hydroxymethyloxalylanilid, N-Hydroxymaleinsäuremonoanilid.

Als Mediatoren werden bevorzugt N-Hydroxyacetanilid, N-Hydroxyformanilid, N-Hydroxy-N-phenyl-methylcarbamat, N-Hydroxy-2-methylacetanilid, N-Hydroxy-4-methylacetanilid, 1-Hydroxy-3,4-dihydrochinolin-(1H)-2-on sowie N-Acetoxyacetanilid.

Der Mediator kann ferner aus der Gruppe der N-Alkyl-N-Hydroxy-A-mide ausgewählt sein.

Bevorzugt werden dabei als Mediatoren Verbindungen der allgemeinen Formel (XVII) oder (XVIII) 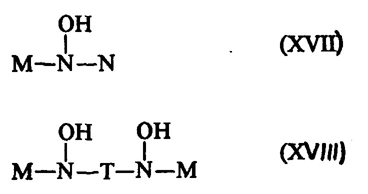 sowie deren Salze, Ether oder Ester eingesetzt, wobei
M gleich oder verschieden ist und einbindiger, linearer oder verzweigter oder cyclischer oder polycyclischer, gesättigter oder ungesättigter Alkylrest mit 1-24 C-Atomen bedeutet
   und
   wobei dieser Alkylrest durch einen oder mehrere Reste R⁴⁵, die gleich oder verschieden sind und ausgewählt sind aus der Gruppe Hydroxy-, Mercapto-, Formyl-, Carbamoyl-, Carboxy-, Ester oder Salz des Carboxyrests, Sulfonorest, Ester oder Salz des Sulfonorests, Sulfamoyl-, Nitro-, Nitroso-, Amino-, Hydroxylamino-, Phenyl-, C₁-C₅-Alkoxy-, C₁-C₁₀-Carbonyl-, Phospho-, Phosphono-, Phosphonooxyrest, Ester oder Salz des Phosphonooxyrests substituiert sein kann und
   wobei Carbamoyl, Sulfamoyl-, Amino-, Hydroxylamino-, Mercapto- und Phenylreste unsubstituiert oder ein- oder mehrfach mit einem Rest R⁴⁶ substituiert sein können und die C₁-C₅-Alkoxy-, C₁-C₁₀-Carbonyl-Reste gesättigt oder ungesättigt, verzweigt oder unverzweigt sein können und mit einem Rest R⁴⁶ ein- oder mehrfach substituiert sein können, wobei
R⁴⁶ gleich oder verschieden ist und Hydroxy-, Formyl-, Cyano-, Carboxyrest, Ester oder Salz des Carboxyrests, Carbamoyl, Sulfono-, Sulfamoyl-, Nitro-, Nitroso-, Amino-, Phenyl-, Benzoyl-, C₁-C₅-Alkyl-, C₁-C₅-Alkoxy-, C₁-C₅-Alkylcarbonylrest bedeutet und nicht α-ständige Methylengruppen durch Sauerstoff, Schwefel oder einen ggf. einfach substituierten Iminorest ersetzt sein können und
N in amidischer Form vorliegender einbindiger Säurerest von Säuren, ausgewählt aus der Gruppe aliphatischer oder ein- oder zweikerniger aromatischer oder ein- oder zweikerniger heteroaromatischer Carbonsäuren mit bis zu 20 C-Atomen, Kohlensäure, Halbester der Kohlensäure oder der Carbaminsäure, Sulfonsäure, Phosphonsäure, Phosphorsäure, Monoester der Phosphorsäure, Diester der Phosphorsäure bedeutet und
T in amidischer Form vorliegender zweibindiger Säurerest von Säuren, ausgewählt aus der Gruppe aliphatischer, ein- oder zweikerniger aromatischer oder ein- oder zweikerniger heteroaromatischer Dicarbonsäuren mit bis zu 20 C-Atomen, Kohlensäure, Sulfonsäure, Phosphonsäure, Phosphorsäure, Monoester der Phosphorsäure bedeutet und
   wobei Alkylreste der in amidischer Form vorliegenden aliphatischen Säuren N und T linear oder verzweigt und/oder cyclisch und/oder polycyclisch, gesättigt oder ungesättigt sein können und null bis 24 Kohlenstoffatome beinhalten und nicht substituiert sind oder ein- oder mehrfach mit dem Rest R⁴⁵ substituiert sind und
Aryl- und Heteroarylreste der in amidischer Form vorliegenden aromatischen oder heteroaromatischen Säuren N und T durch einen oder mehrere Reste R⁴⁷, die gleich oder verschieden sind und ausgewählt sind aus der Gruppe Hydroxy-, Mercapto-, Formyl-, Cyano-, Carbamoyl-, Carboxy-, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-Alkyl-, C₁-C₁₂-Alkyl-, C₁-C₅-Alkoxy-, C₁-C₁₀-Carbonyl-, Phospho-, Phosphono-, Phosphonooxyrest, Ester oder Salz des Phosphonooxyrests substituiert sein können und
   wobei Carbamoyl, Sulfamoyl-, Amino-, Mercapto- und Phenylreste unsubstituiert oder ein- oder mehrfach mit dem Rest R⁴⁶ substituiert sein können und die Aryl-C₁-C₅-Alkyl-, C₁-C₁₂-Alkyl-C₁-C₅-Alkoxy-, C₁-C₁₀-Carbonyl-Reste gesättigt oder ungesättigt, verzweigt oder unverzweigt sein können und ein- oder mehrfach mit dem Rest R⁴⁶ substituiert sein können.

Als Mediatoren besonders bevorzugt sind Verbindungen mit den allgemeinen Formeln (XIX), (XX) , (XXI) oder (XXII): 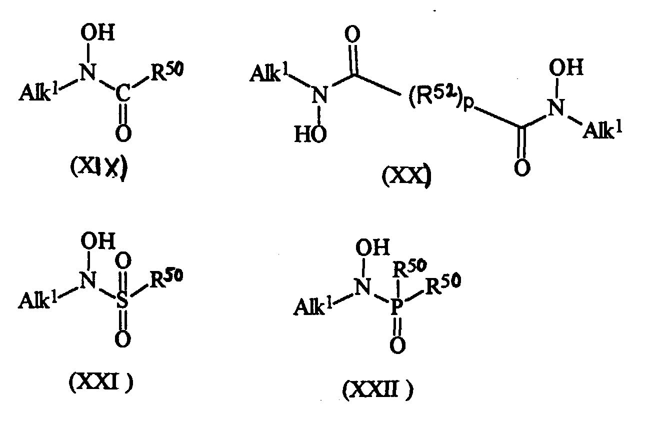 sowie deren Salze, Ether oder Ester, wobei
Alk¹ gleich oder verschieden ist und einbindiger, linearer oder verzweigter oder cyclischer oder polycyclischer, gesättigter oder ungesättigter Alkylrest mit 1-10 C-Atomen bedeutet,
   wobei dieser Alkylrest durch einen oder mehrere Reste R⁴⁸, die gleich oder verschieden sind und ausgewählt sind aus der Gruppe Hydroxy-, Formyl-, Carbamoyl-, Carboxy-, Ester oder Salz des Carboxyrests, Sulfonorest, Ester oder Salz des Sulfonorests, Sulfamoyl-, Nitro-, Nitroso-, Amino-, Hydroxylamino-, Phenyl-, C₁-C₅-Alkoxy-, C₁-C₅-Carbonyl-Reste substituiert sein kann und wobei Carbamoyl, Sulfamoyl-, Amino-, Hydroxylamino- und Phenylreste unsubstituiert oder ein- oder mehrfach mit einem Rest R⁴⁹ substituiert sein können und die C₁-C₅-Alkoxy-, C₁-C₁₀-Carbonyl-Reste gesättigt oder ungesättigt, verzweigt oder unverzweigt sein können und mit einem Rest R⁴⁹ ein- oder mehrfach substituiert sein können, wobei
R⁴⁹ gleich oder verschieden ist und Hydroxy-, Formyl-, Cyano-, Carboxyrest, Ester oder Salz des Carboxyrests, Carbamoyl, Sulfono-, Sulfamoyl-, Nitro-, Amino-, Phenyl-, Benzoyl-, C₁-C₅-Alkyl-, C₁-C₅-Alkoxy-, C₁-C₅-Alkylcarbonylrest bedeutet und
   nicht α-ständige Methylengruppen durch Sauerstoff, Schwefel oder einen ggf. einfach substituierten Iminorest ersetzt sein können und
   wobei R⁵⁰ gleiche oder verschiedene einbindige Reste, ausgewählt aus der Gruppe Wasserstoff-, Phenyl-, Pyridyl-, Furyl-, Pyrrolyl-, Thienyl-, Aryl-C₁-C₅-Alkyl-, C₁-C₁₂-Alkyl-, C₁-C₁₀-Alkoxy-, C₁-C₁₀-Carbonylrest bedeutet,
   wobei Phenyl-, Pyridyl-, Furyl-, Pyrrolyl- und Thienylreste unsubstituiert oder ein- oder mehrfach mit einem Rest R⁵¹ substituiert sein können und die Aryl-C₁-C₅-alkyl-, C₁-C₁₂-Alkyl-, C₁-C₅-Alkoxy- und C₁-C₁₀-Carbonyl-Reste gesättigt oder ungesättigt, verzweigt oder unverzweigt sein können und mit einem Rest R⁵¹ einoder mehrfach substituiert sein können und
R⁵¹ gleich oder verschieden ist und Hydroxy-, Formyl-, Carboxyrest, Ester oder Salz des Carboxyrests, Carbamoyl-, Sulfono-, Sulfamoyl-, Nitro-, Amino-, Phenyl-, C₁-C₅-Alkyl-, C₁-C₅-Alkoxyrest bedeutet und
R⁵² zweibindige Reste, ausgewählt aus der Gruppe Phenylen-, Pyridylen-, Thienylen-, Furylen-, Pyrrolylen-, Aryl-C₁-C₅-alkyl-, C₁-C₁₂-Alkylen-, C₁-C₅-Alkylendioxy-Rest bedeutet, wobei Phenylen-, Pyridylen-, Thienylen-, Furylen-, Pyrrolylen- unsubstituiert oder ein- oder mehrfach mit einem Rest R⁵³ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁵¹ ein- oder mehrfach substituiert sein können, wobei
p 0 oder 1 bedeutet.

Als Mediatoren ganz besonders bevorzugt sind Verbindungen mit der allgemeinen Formel (XIX) - (XXII), bei denen
Alk¹ gleich oder verschieden ist und einbindiger, linearer oder verzweigter oder cyclischer, gesättigter oder ungesättigter Alkylrest mit 1-10 C-Atomen bedeutet,
   wobei dieser Alkylrest durch einen oder mehrere Reste R⁴⁸, die gleich oder verschieden sind und ausgewählt sind aus der Gruppe Hydroxy-, Carbamoyl-, Carboxy-, Ester oder Salz des Carboxyrests, Sulfonorest, Ester oder Salz des Sulfonorests, Sulfamoyl-, Amino-, Phenyl-, C₁-C₅-Alkoxy-, C₁-C₅-Carbonyl-Reste substituiert sein kann und
   wobei Carbamoyl, Sulfamoyl-, Amino- und Phenylreste unsubstituiert oder ein- oder mehrfach mit einem Rest R⁴⁹ substituiert sein können und die C₁-C₅-Alkoxy-, C₁-C₁₀-Carbonyl-Reste gesättigt oder ungesättigt, verzweigt oder unverzweigt sein können und mit einem Rest R⁴⁹ ein- oder mehrfach substituiert sein können, wobei
R⁴⁹ gleich oder verschieden ist und Hydroxy-, Carboxyrest, Ester oder Salz des Carboxyrests, Carbamoyl, Sulfono-, Sulfamoyl-, Nitro-, Amino-, Phenyl-, Benzoyl-, C₁-C₅-Alkyl-, C₁-C₅-Alkoxy-, C₁-C₅-Alkylcarbonylrest bedeutet
   und
   wobei R⁵⁰ gleiche oder verschiedene einbindige Reste, ausgewählt aus der Gruppe Wasserstoff-, Phenyl-, Furyl-, Aryl-C₁-C₅-Alkyl-, C₁-C₁₂-Alkyl-, C₁-C₁₀-Alkoxy-, C₁-C₁₀-Carbonylrest bedeutet,
   wobei Phenyl- und Furylreste unsubstituiert oder ein- oder mehrfach mit einem Rest R⁵¹ substituiert sein können und die Aryl-C₁-C₅-alkyl-, C₁-C₁₂-Alkyl-, C₁-C₅-Alkoxy- und C₁-C₁₀-Carbonyl-Reste gesättigt oder ungesättigt, verzweigt oder unverzweigt sein können und mit einem Rest R⁵¹ ein- oder mehrfach substituiert sein können,
   wobei
R⁵¹ gleich oder verschieden ist und Carboxyrest, Ester oder Salz des Carboxyrests, Carbamoyl-, Phenyl-, C₁-C₅-Alkyl-, C₁-C₅-Alkoxyrest bedeutet und
R⁵² zweibindiger Rest ausgewählt aus der Gruppe Phenylen-, Furylen-, C₁-C₁₂-Alkylen-, C₁-C₅-Alkylendioxy-Rest bedeutet, wobei Phenylen-, Furanylen- unsubstituiert oder ein- oder mehrfach mit einem Rest R⁵¹ substituiert sein können und die Aryl-C₁-C₅-alkyl-, C₁-C₁₂-Alkyl-, C₁-C₅-Alkoxy-Reste gesättigt oder ungesättigt, verzweigt oder unverzweigt sein können und mit einem Rest R⁵¹ ein- oder mehrfach substituiert sein können, wobei
p 0 oder 1 bedeutet.

Beispiele für Verbindungen, die als Mediatoren eingesetzt werden können, sind
N-Hydroxy-N-methyl-benzoesäureamid, N-Hydroxy-N-methyl-benzolsulfonsäure-amid, N-Hydroxy-N-methyl-p-toluolsulfonsäureamid, N-Hydroxy-N-methyl-furan-2-carbonsäureamid, N-Hydroxy-N-methyl-thiophen-2-carbonsäureamid, N,N'-Dihydroxy-N,N'-dimethyl-phthalsäurediamid, N,N'-Dihydroxy-N,N'-dimethyl-isophthalsäurediamid, N,N'-Dihydroxy-N,N'-dimethyl-terephthalsäurediamid, N,N'-Dihydroxy-N,N'-dimethyl-benzol-1,3-disulfonsäurediamid, N,N'-Dihydroxy-N,N'-dimethyl-furan-3,4-dicarbonsäurediamid, N-Hydroxy-N-tert.-butyl-benzoesäureamid, N-Hydroxy-N-tert.-butyl-benzolsulfonsäureamid, N-Hydroxy-N-tert.-butyl-p-toluolsulfonsäureamid, N-Hydroxy-N-tert.-butyl-furan-2-carbonsäureamid, N-Hydroxy-N-tert.-butyl-thiophen-2-carbonsäureamid, N,N'-Dihydroxy-N,N'-di-tert.-butyl-phthalsäurediamid, N,N'-Dihydroxy-N'N'-di-tert.-butyl-isophthalsäurediamid, N,N'-Dihydroxy-N,N'-di-tert.-butyl-terephthalsäurediamid, N,N'-Dihydroxy-N,N'-di-tert.-butyl-benzol-1,3-disulfonsäurediamid, N,N'-Dihydroxy-N,N'-di-tert.-butyl-furan-3,4-dicarbonsäurediamid, N-Hydroxy-N-cyclohexyl-benzoesäureamid, N-Hydroxy-N-cyclohexylbenzolsulfonsäureamid, N-Hydroxy-N-cyclohexyl-p-toluolsulfonsäure-amid, N-Hydroxy-N-cyclohexyl-furan-2-carbonsäureamid, N-Hydroxy-N-cyclohexyl-thiophen-2-carbonsäureamid, N,N'-Dihydroxy-N,N'-dicyclohexyl-phthalsäurediamid, N,N'-Dihydroxy-N,N'-dicyclohexyl-isophthalsäurediamid, N,N'-Dihydroxy-N,N'-dicyclohexyl-terephthalsäurediamid, N,N'-Dihydroxy-N,N'-dicyclohexyl-benzol-1,3-disulfonsäurediamid, N,N'-Dihydroxy-N,N'-dicyclohexyl-furan-3,4-dicarbonsäure-diamid, N-Hydroxy-N-isopropyl-benzoesäureamid, N-Hydroxy-N-isopropyl-benzol-sulfonsäureamid, N-Hydroxy-N-isopropyl-p-toluol-sulfonsäureamid,N-Hydroxy-N-isopropyl-furan-2-carbonsäureamid, N-Hydroxy-N-isopropyl-thiophen-2-carbon-säureamid, N,N'-Dihydroxy-N,N'-diisopropyl-phthalsäurediamid, N,N'-Dihydroxy-N,N'-diisopropyl-isophthalsäurediamid, N,N'-Dihydroxy-N,N'-diisopropyl-terephthal-säurediamid, N,N'-Dihydroxy-N,N'-diisopropyl-benzol-1,3-disulfonsäurediamid, N,N'-Dihydroxy-N,N'-diisopropyl-furan-3,4-dicarbonsäurediamid, N-Hydroxy-N-methyl-acetamid, N-Hydroxy-N-tert.-butyl-acetamid, N-Hydroxy-N-isopropyl-acetamid, N-Hydroxy-N-cyclohexyl-acetamid, N-Hydroxy-N-methyl-pivalinsäureamid, N-Hydroxy-N-isopropyl-pivalinsäureamid, N-Hydroxy-N-methyl-acrylamid, N-Hydroxy-N-tert.-butyl-acrylamid, N-Hydroxy-N-isopropyl-acrylamid, N-Hydroxy-N-cyclohexyl-acrylamid, N-Hydroxy-N-methyl-methansulfonamid, N-Hydroxy-N-isopropyl-methansulfonamid, N-Hydroxy-N-isopropyl-methylcarbamat, N-Hydroxy-N-methyl-3-oxo-buttersäureamid, N,N'-Dihydroxy-N,N'-dibenzoyl-ethylendiamin, N,N'-Dihydroxy-N,N'-dimethyl-bernsteinsäurediamid, N,N'-Dihydroxy-N,N'-di-tert.-butyl-maleinsäurediamid, N-Hydroxy-N-tert.-butyl-maleinsäuremonoamid, N,N'-Dihydroxy-N,N'-di-tert.-butyl-oxalsäurediamid, N,N'-Dihydroxy-N,N'-di-tert.-butyl-phosphor-säurediamid.

Als Mediatoren werden bevorzugt Verbindungen ausgewählt aus der Gruppe N-Hydroxy-N-methyl-benzoesäureamid, N-Hydroxy-N-methyl-benzolsulfonsäureamid, N-Hydroxy-N-methyl-p-toluolsulfon-säureamid, N-Hydroxy-N-methyl-furan-2-carbonsäureamid, N,N'-Dihydroxy-N,N'-dimethyl-phthalsäurediamid, N,N'-Dihydroxy-N,N'-dimethyl-terephthalsäurediamid, N,N'-Dihydroxy-N,N'-dimethyl-benzol-1,3-disulfonsäurediamid, N-Hydroxy-N-tert.-butyl-benzoesäureamid, N-Hydroxy-N-tert.-butyl-benzolsulfonsäureamid, N-Hydroxy-N-tert.-butyl-p-toluolsulfonsäureamid, N-Hydroxy-N-tert.-butyl-furan-2-carbonsäureamid, N,N'-Dihydroxy-N,N'-di-tert.-butyl-terephthalsäurediamid, N-Hydroxy-N-isopropyl-benzoesäureamid, N-Hydroxy-N-isopropyl-p-toluolsulfon-säureamid, N-Hydroxy-N-isopropyl-furan-2-carbonsäureamid, N,N'-Dihydroxy-N,N'-diisopropyl-terephthal-säurediamid, N,N'-Dihydroxy-N,N'-diisopropyl-benzol-1,3-disulfonsäurediamid, N-Hydroxy-N-methyl-acetamid, N-Hydroxy-N-tert.-butyl-acetamid, N-Hydroxy-N-isopropyl-acetamid, N-Hydroxy-N-cyclohexyl-acetamid, N-Hydroxy-N-methyl-pivalinsäureamid, N-Hydroxy-N-tert.-butyl-acrylamid, N-Hydroxy-N-isopropyl-acrylamid, N-Hydroxy-N-methyl-3-oxo-buttersäureamid, N,N'-Dihydroxy-N,N'-dibenzoyl-ethylendiamin, N,N'-Dihydroxy-N,N'-di-tert.-butyl-maleinsäurediamid, N-Hydroxy-N-tert.-butyl-maleinsäuremonoamid, N,N'-Dihydroxy-N,N'-di-tert.-butyl-oxalsäurediamid.

Der Mediator kann ferner ausgewählt sein aus der Gruppe der Oxime der allgemeinen Formel XXIII oder XXIV 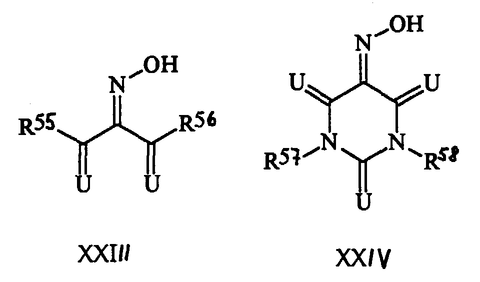 sowie deren Salze, Ether, oder Ester, wobei
U gleich oder verschieden ist und O, S, oder NR⁵³ bedeutet, wobei
R⁵³ Wasserstoff-, Hydroxy-, Formyl-, Carbamoyl-, Sulfonorest, Ester oder Salz des Sulfonorests, Sulfamoyl-, Nitro-, Amino-, Phenyl-, Aryl-C₁- C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphonooxyrests bedeutet,
   wobei Carbamoyl-, Sulfamoyl-, Amino- und Phenylreste unsubstituiert oder ein- oder mehrfach mit einem Rest R⁵⁴ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁵⁴ ein- oder mehrfach substituiert sein können, wobei
R⁵⁴ gleich oder verschieden ist und Hydroxy-, Formyl-, Carboxyrest, Ester oder Salz des Carboxyrests, Carbamoyl-, Sulfono-Ester oder Salz des Sulfonorests, Sulfamoyl-, Nitro-, Amino-, Phenyl-, C₁-C₅-Alkyl-, C₁-C₅-Alkoxyrest bedeutet und
die Reste R⁵⁵ und R⁵⁶ gleich oder verschieden sind und Halogen-, Carboxyrest, Ester oder Salz des Carboxyrests bedeuten, oder die für R⁵³ genannten Bedeutungen haben, oder zu einem Ring [-CR⁵⁹R⁶⁰]ₙ mit n gleich 2, 3 oder 4 verknüpft sind und
R⁵⁷ und R⁵⁸ die für R⁵³ genannten Bedeutungen haben und
R⁵⁹ und R⁶⁰ gleich oder verschieden sind und Halogen-, Carboxyrest, Ester oder Salz des Carboxyrests bedeuten, oder die für R⁵³ genannten Bedeutungen haben.

Als Mediatoren besonders bevorzugt sind Verbindungen mit der allgemeinen Formel XXIII bei denen U O oder S bedeutet und die übrigen Reste die vorstehend genannten Bedeutungen haben. Ein Beispiel für eine solche Verbindung ist 2-Hydroxyiminomalonsäuredimethylester.

Als Mediatoren weiterhin besonders bevorzugt sind Isonitrosoderivate von cyclischen Ureiden der allgemeinen Formel XXIV. Beispiele für solche Verbindungen sind 1-Methylviolursäure, 1,3-Dimethylviolursäure, Thioviolursäure, Alloxan-4,5-dioxim.

Als Mediator insbesondere bevorzugt ist Alloxan-5-oxim Hydrat (Violursäure) und/oder dessen Ester, Ether oder Salze.

Der Mediator kann ferner ausgewählt sein aus der Gruppe vicinal nitrososubstituierter aromatischer Alkohole der allgemeinen Formel XXV oder XXVI 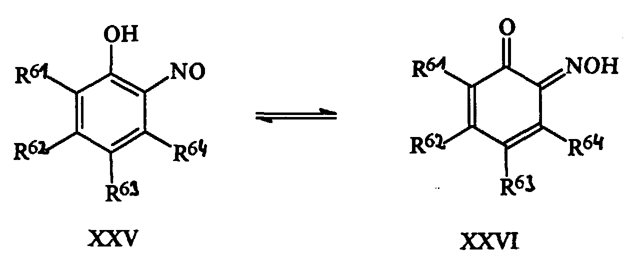 sowie deren Salze, Ether oder Ester, wobei
R⁶¹, R⁶², R⁶³ und R⁶⁴ gleich oder verschieden sind und Wasserstoff-, Halogen-, Hydroxy-, Formyl-, Carbamoyl-, Carboxyrest, Ester oder Salz des Carboxyrests, Sulfonorest, Ester oder Salz des Sulfonorests, Sulfamoyl-, Nitro-, Nitroso-, Cyano, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests bedeuten,
   wobei Carbamoyl-, Sulfamoyl-, Amino- und Phenylreste unsubstituiert oder ein- oder mehrfach mit einem Rest R⁶⁵ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁶⁵ ein- oder mehrfach substituiert sein können, wobei
R⁶⁵ gleich oder verschieden ist und Hydroxy-, Formyl-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁-C₅-Alkoxyrest bedeutet oder
die Reste R⁶¹-R⁶⁴ paarweise zu einem Ring [-CR⁶⁶R⁶⁷-]ₘ verknüpft sind, wobei m ganzzahlig ist und einen Wert von 1 bis 4 bedeutet, oder zu einem Ring [-CR⁶⁸=CR⁶⁹-]ₙ verknüpft sind, wobei n ganzzahlig ist und einen Wert von 1 bis 3 bedeutet, und
R⁶⁶, R⁶⁷, R⁶⁸ und R⁶⁹ gleich oder verschieden sind und die für R⁶¹ bis R⁶⁴ genannten Bedeutungen haben.

Unter aromatischen Alkoholen sind vorzugsweise Phenole oder höherkondensierte Derivate des Phenols zu verstehen.

Als Mediatoren bevorzugt sind Verbindungen der allgemeinen Formel XXV oder XXVI, deren Synthese sich auf die Nitrosierung substituierter Phenole zurückführen läßt. Beispiele für solche Verbindungen sind 2-Nitrosophenol, 3-Methyl-6-nitrosophenol, 2-Methyl-6-nitrosophenol, 4-Methyl-6-nitrosophenol, 3-Ethyl-6-nitrosophenol, 2-Ethyl-6-nitrosophenol, 4-Ethyl-6-nitrosophenol, 4-Isopropyl-6-nitrosophenol, 4-tert.butyl-6-nitrosophenol, 2-Phenyl-6-nitrosophenol, 2-Benzyl-6-nitrosophenol, 4-Benzyl-6-nitrosophenol, 2-Hydroxy-3-nitrosobenzylalkohol, 2-Hydroxy-3-nitrosobenzoesäure, 4-Hydroxy-3-nitrosobenzoesäure, 2-Methoxy-6-nitrosophenol, 3,4-Dimethyl-6-nitrosophenol, 2,4-Dimethyl-6-nitrosophenol, 3,5-Dimethyl-6-nitrosophenol, 2,5-Dimethyl-6-nitrosophenol, 2-Nitrosoresorcin, 4-Nitrosoresorcin, 2-Nitrosoresorcin, 2-Nitrosophloroglucin und 4-Nitrosopyrogallol, 4-Nitroso-3-hydroxyanilin, 4-Nitro-2-nitrosophenol.

Als Mediatoren weiterhin bevorzugt sind o-Nitrosoderivate höher kondensierter aromatischer Alkohole. Beispiele für solche Verbindungen sind 2-Nitroso-1-naphthol, 1-Methyl-3-nitroso-2-naphthol und 9-Hydroxy-10-nitroso-phenanthren.

Als Mediatoren besonders bevorzugt sind 1-Nitroso-2-naphthol, 1-Nitroso-2-naphthol-3,6-disulfonsäure, 2-Nitroso-1-naphthol-4-sulfonsäure, 2,4-Dinitroso-1,3-dihydroxybenzol sowie Ester, Ether oder Salze der genannten Verbindungen.

Der Mediator kann ferner ausgewählt sein aus der Gruppe Hydroxypyridine, Aminopyridine, Hydroxychinoline, Aminochinoline, Hydroxyisochinoline, Aminoisochinoline mit zu den Hydroxy- oder Aminogruppen ortho- oder para-ständigen Nitroso- oder Mercaptosubstituenten, Tautomere der genannten Verbindungen sowie deren Salze, Ether und Ester.

Bevorzugt sind als Mediatoren Verbindungen der allgemeinen Formel (XXVII), (XXVIII) oder (XXIX) 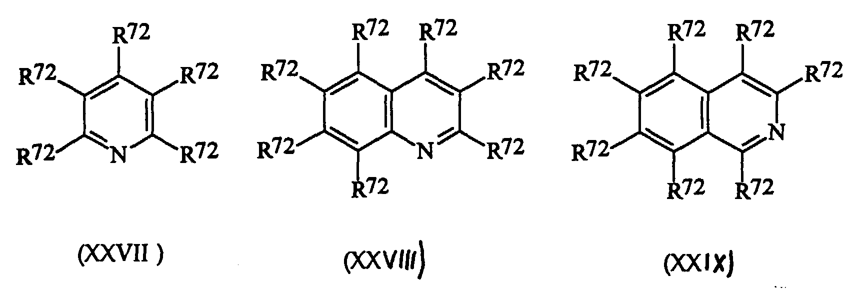 sowie Tautomere, Salze, Ether oder Ester der genannten Verbindungen vorhanden, wobei in den Formeln XXVII, XXVIII und XXIX zwei zueinander ortho- oder para- ständige Reste R⁷⁰ Hydroxy- und Nitrosorest oder Hydroxy- und Mercaptorest oder Nitrosorest und Aminorest bedeuten
und die übrigen Reste R⁷⁰ gleich oder verschieden sind und ausgewählt sind aus der Gruppe Wasserstoff-, Halogen-, Hydroxy-, Mercapto-, Formyl-, Cyano-, Carbamoyl-, Carboxyrest, Ester und Salz des Carboxyrests, Sulfonorest, Ester und Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy, C₁-C₁₀-Carbonyl-, Carbonyl-C₁-C₆-alkyl-, Phospho-, Phosphono-, Phosphono-oxyrest, Ester und Salz des Phosphonooxyrests und
   wobei Carbamoyl-, Sulfamoyl-, Amino-, Mercapto- und Phenylreste unsubstituiert oder ein- oder mehrfach mit einem Rest R⁷¹ substituiert sein können und
die Aryl-C₁-C₅-alkyl-, C₁-C₁₂-Alkyl-, C₁-C₅-Alkoxy-, C₁-C₁₀-Carbonyl-, Carbonyl-C₁-C₆-alkylreste gesättigt oder ungesättigt, verzweigt oder unverzweigt sein können und mit einem Rest R⁷¹ ein- oder mehrfach substituiert sein können, wobei R⁷¹ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁-C₅-Alkoxyrest oder C₁-C₅-Alkylcarbonylrest bedeutet und
je zwei Reste R⁷⁰ oder zwei Reste R⁷¹ oder R⁷⁰ und R⁷¹ paarweise über eine Brücke [-CR⁷²R⁷³-]ₘ mit m gleich 1,2, 3 oder 4 verknüpft sein können und
R⁷² und R⁷³ gleich oder verschieden sind und Carboxyrest, Ester oder Salz des Carboxyrests, Phenyl-, C₁-C₅-Alkyl-, C₁-C₅-Alkoxyrest oder C₁-C₅-Alkylcarbonylrest bedeuten und eine oder mehrere nicht benachbarte Gruppen [-CR⁷²R⁷³-] durch Sauerstoff, Schwefel oder einen ggf. mit C₁-C₅-Alkyl- substituierten Iminorest und zwei benachbarte Gruppen [-CR⁷²R⁷³-] durch eine Gruppe [-CR⁷²=R⁷³-] ersetzt sein können.

Als Mediatoren besonders bevorzugt sind Verbindungen der allgemeinen Formel (XXVII) oder (XXVIII) sowie deren Tautomere, Salze, Ether oder Ester, wobei in den Formeln (XXVII) und (XXVIII) besonders bevorzugt zwei zueinander ortho- ständige Reste R⁷⁰ Hydroxy- und Nitrosorest oder Hydroxy- und Mercaptorest oder Nitrosorest- und Aminorest bedeuten und
die übrigen Reste R⁷⁰ gleich oder verschieden sind und ausgewählt sind aus der Gruppe Wasserstoff-, Hydroxy-, Mercapto-, Formyl-, Carbamoyl-, Carboxyrest, Ester und Salz des Carboxyrests, Sulfonorest, Ester und Salz des Sulfonorests, Sulfamoyl-, Nitro-, Nitroso-, Amino-, Phenyl-, Aryl-C₁-C₅-alkyl-, C₁-C₅-Alkyl-, C₁-C₅-Alkoxy-, C₁-C₅-Carbonyl-, Carbonyl-C₁-C₆-alkyl-, Phospho-, Phosphono-, Phosphono-oxyrest, Ester und Salz des Phosphonooxyrests wobei
Carbamoyl-, Sulfamoyl-, Amino-, Mercapto- und Phenylreste unsubstituiert oder ein- oder mehrfach mit einem Rest R⁷¹ substituiert sein können und
die Aryl-C₁-C₅-alkyl-, C₁-C₅-Alkyl-, C₁-C₅-Alkoxy-, C₁-C₅-Carbonyl-, Carbonyl-C₁-C₆-alkyl-Reste gesättigt oder ungesättigt, verzweigt oder unverzweigt sein können und mit einem Rest R⁷¹ ein- oder mehrfach substituiert sein können, wobei
R⁷¹ die bereits genannten Bedeutungen hat und
je zwei Reste R⁷¹ paarweise über eine Brücke [-CR⁷²R⁷³-]ₘ mit m gleich 2, 3 oder 4 verknüpft sein können und
R⁷² und R⁷³ die bereits genannten Bedeutungen haben und eine oder mehrere nicht benachbarte Gruppen [-CR⁷²R⁷³-] durch Sauerstoff oder einen ggf. mit C₁-C₅-Alkyl- substituierten Iminorest ersetzt sein können.

Beispiele für Verbindungen, die als Mediatoren eingesetzt werden können, sind 2,6-Dihydroxy-3-nitrosopyridin, 2,3-Dihydroxy-4-nitrosopyridin, 2,6-Dihydroxy-3-nitrosopyridin-4-carbonsäure, 2,4-Dihydroxy-3-nitrosopyridin, 3-Hydroxy-2-mercaptopyridin, 2-Hydroxy-3-mercaptopyridin, 2,6-Diamino-3-nitrosopyridin, 2,6-Diamino-3-nitroso-pyridin-4-carbonsäure, 2-Hydroxy-3-nitrosopyridin, 3-Hydroxy-2-nitrosopyridin, 2-Mercapto-3-nitrosopyridin, 3-Mercapto-2-nitrosopyridin, 2-Amino-3-nitrosopyridin, 3-Amino-2-nitrosopyridin, 2,4-Dihydroxy-3-nitrosochinolin, 8-Hydroxy-5-nitrosochinolin, 2,3-Dihydroxy-4-nitrosochinolin, 3-Hydroxy-4-nitrosoisochinolin, 4-Hydroxy-3-nitrosoisochinolin, 8-Hydroxy-5-nitrosoisochinolin sowie Tautomere dieser Verbinungen.

Als Mediatoren sind bevorzugt 2,6-Dihydroxy-3-nitrosopyridin, 2,6-Diamino-3-nitrosopyridin, 2,6-Dihydroxy-3-nitrosopyridin-4-carbonsäure, 2,4-Dihydroxy-3-nitrosopyridin, 2-Hydroxy-3-mercaptopyridin, 2-Mercapto-3-pyridinol, 2,4-Dihydroxy-3-nitrosochinolin, 8-Hydroxy-5-nitrosochinolin, 2,3-Dihydroxy-4-nitrosochinolin sowie Tautomere dieser Verbindungen.

Der Mediator kann ferner aus der Gruppe stabiler Nitroxyl-Radikale (Nitroxide) ausgewählt sein, d.h. diese freien Radikale können in reiner Form erhalten, charakterisiert und aufbewahrt werden.

Bevorzugt werden dabei als Mediatoren Verbindungen der allgemeinen Formel (XXX), (XXXI) oder (XXXII) eingesetzt 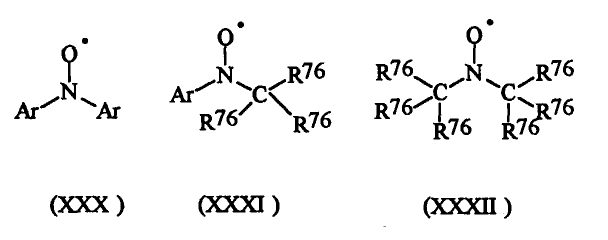 wobei
Ar einbindiger homo- oder heteroaromatischer ein- oder zweikerniger Rest bedeutet und
   wobei dieser aromatische Rest durch einen oder mehrere, gleiche oder verschiedene Reste R⁷⁵ , ausgewählt aus der Gruppe Halogen-, Formyl-, Cyano-, Carbamoyl-, Carboxy-, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-Alkyl-, C₁-C₁₂-Alkyl-, C₁-C₅-Alkoxy-, C₁-C₁₀-Carbonyl-, Carbonyl-C₁-C₆-Alkyl-, Phospho-, Phosphono-, Phosphonooxyrest, Ester oder Salz des Phosphonooxyrests substituiert sein können
   und
   wobei Phenyl-, Carbamoyl- und Sulfamoylreste unsubstituiert oder ein- oder mehrfach mit einem Rest R⁷⁶ substituiert sein können, der Aminorest ein- oder zweifach mit R⁷⁶ substituiert sein kann und die Aryl-C₁-C₅-alkyl, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁷⁶ ein- oder mehrfach substituiert sein können, wobei
R⁷⁶ein- oder mehrfach vorhanden sein kann und gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁-C₅-Alkoxy-, C₁-C₅-Alkylcarbonylrest bedeutet
   und
R⁷⁴ gleich oder verschieden ist und Halogen-, Hydroxy-, Mercapto-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-,
C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests bedeutet
und R⁷⁴ im Fall bicyclischer stabiler Nitroxylradikale (Struktur XXXII) auch Wasserstoff bedeuten kann
   und
   wobei Carbamoyl-, Sulfamoyl-, Amino-, Mercapto- und Phenylreste unsubstituiert oder ein- oder mehrfach mit einem Rest R⁷⁷ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R₇₇ ein- oder mehrfach substituiert sein können, wobei R⁷⁷ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino, Phenyl-, C₁-C₅-Alkyl-, C₁ - C₅-Alkoxyrest, C₁ - C₅-Alkylcarbonylrest bedeutet und
je zwei Reste R⁷⁶oder R⁷⁷ paarweise über eine Brücke [-CR⁷⁸R⁷⁹-]ₘmit m gleich 0,1,2,3 oder 4 verknüpft sein können
   und
R⁷⁸ und R⁷⁹ gleich oder verschieden sind und Halogen-, Carboxyrest, Ester oder Salz des Carboxyrests, Carbamoyl-, Sulfamoyl-, Phenyl-, Benzoyl-, C₁-C₉-Alkyl-, C₁-C₅-Alkoxyrest, C₁-C₅-Alkylcarbonylrest bedeuten und
eine oder mehrere nicht benachbarte Gruppen [-CR⁷⁸R⁷⁹-] durch Sauerstoff, Schwefel oder einen ggf. mit C₁-C₅-Alkyl- substituierten Iminorest und zwei benachbarte Gruppen [-CR⁷⁸R⁷⁹-] durch eine Gruppe [-CR⁷⁸=CR⁷⁹-], [-CR⁷⁸=N-] oder [-CR⁷⁸=N(O)-] ersetzt sein können.

Als Mediatoren besonders bevorzugt sind Nitroxyl-Radikale der allgemeinen Formeln (XXXIII) und (XXXIV), 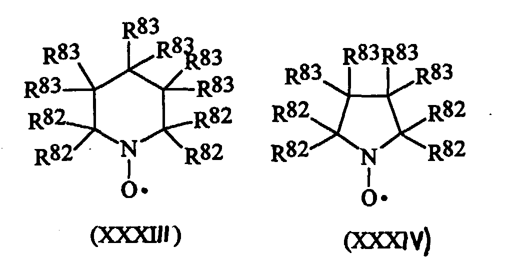 wobei
R⁸⁰ gleich oder verschieden ist und Phenyl-, Aryl-C₁-C₅-alkyl-, C₁-C₁₂-Alkyl-, C₁-C₅-Alkoxy,-, C₁-C₁₀-Carbonyl-, Carbonyl-C₁-C₆-alkyl-Rest bedeutet
   wobei Phenylreste unsubstituiert oder ein- oder mehrfach mit einem Rest R⁸² substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁸² ein- oder mehrfach substituiert sein können,
   wobei R⁸² ein- oder mehrfach vorhanden sein kann und gleich oder verschieden ist und Hydroxy-, Formyl-, Carboxyrest, Ester oder Salz des Carboxyrests, Carbamoyl-, Sulfono-, Sulfamoyl-, Nitro-, Nitroso-, Amino, Phenyl-, Benzoyl-, C₁-C₅-Alkyl-, C₁ - C₅-Alkoxyrest, C₁ - C₅-Alkylcarbonylrest bedeutet und
R⁸¹ gleich oder verschieden ist und Wasserstoff-, Hydroxy-, Mercapto-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphonooxyrest, Ester oder Salz des Phosphono-oxyrests bedeutet
   wobei Carbamoyl-, Sulfamoyl-, Amino-, Mercapto- und Phenylreste unsubstituiert oder ein- oder mehrfach mit einem Rest R⁷⁶ substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁷⁶ ein- oder mehrfach substituiert sein können und eine [-CR⁸¹R⁸¹-] -Gruppe durch Sauerstoff, einen ggf. mit C₁-C₅-Alkyl-substituierten Iminorest, einen (Hydroxy)iminorest, eine Carbonylfunktion oder eine ggf. mit R⁷⁶ mono- oder disubstituierten Vinylidenfunktion ersetzt sein kann und
zwei benachbarte Gruppen [-CR⁸¹R⁸¹-] durch eine Gruppe [-CR⁸¹=CR⁸¹-] oder [-CR⁸¹=N-] oder [-CR⁸¹=N(O)-] ersetzt sein können.

Beispiele für Verbindungen, die als Mediatoren eingesetzt werden können, sind
2,2,6,6-Tetramethyl-piperidin-1-oxyl (TEMPO), 4-Hydroxy-2,2,6,6-tetramethyl-piperidin-1-oxyl, 4-Oxo-2,2,6,6-tetramethyl-piperidin-1-oxyl, 4-Acetamido-2,2,6,6-tetramethyl-piperidin-1-oxyl, 4-(Ethoxyfluorphosphinyloxy)-2,2,6,6-tetramethyl-piperidin-1-oxyl, 4-(Isothiocyanato)-2,2,6,6-tetramethyl-piperidin-1-oxyl, 4-Maleimido-2,2,6,6-tetramethyl-piperidin-1-oxyl, 4-(4-Nitrobenzoyloxy)-2,2,6,6-tetramethyl-piperidin-1-oxyl, 4-(Phosphonooxy)-2,2,6,6-tetramethyl-piperidin-1-oxyl, 4-Cyano-2,2,6,6-tetramethyl-piperidin-1-oxyl, 3-Carbamoyl-2,2,5,5-tetramethyl-3-pyrrolin-1-oxyl, 4-Phenyl-2,2,5,5-tetramethyl-3-imidazolin-3-oxid-1-oxyl, 4-Carbamoyl-2,2,5,5-tetramethyl-3-imidazolin-3-oxid-1-oxyl, 4-Phenacyliden-2,2,5,5-tetramethyl-imidazolidin-1-oxyl, 3-(Aminomethyl)-2,2,5,5-tetramethyl-pyrrolidin-N-oxyl, 3-Carbamoyl-2,2,5,5-tetramethyl-pyrrolidin-N-oxyl, 3-Carboxy-2,2,5,5-tetramethyl-pyrrolidin-N-oxyl, 3-Cyano-2,2,5,5-tetramethyl-pyrrolidin-N-oxyl, 3-Maleimido-2,2,5,5-tetramethyl-pyrrolidin-N-oxyl, 3-(4-Nitrophenoxycarbonyl)-2,2,5,5-tetramethyl-pyrrolidin-N-oxyl.

Als Mediatoren werden bevorzugt
2,2,6,6-Tetramethyl-piperidin-1-oxyl (TEMPO),
4-Hydroxy-2,2,6,6-tetramethyl-piperidin-1-oxyl,
4-Oxo-2,2,6,6-tetramethyl-piperidin-1-oxyl,
4-Acetamido-2,2,6,6-tetramethyl-piperidin-1-oxyl,
4-(Isothiocyanato)-2,2,6,6-tetramethyl-piperidin-1-oxyl,
4-Maleimido-2,2,6,6-tetramethyl-piperidin-1-oxyl,
4-(4-Nitrobenzoyloxy)-2,2,6,6-tetramethyl-piperidin-1-oxyl,
4-(Phosphonooxy)-2,2,6,6-tetramethyl-piperidin-1-oxyl,
4-Cyano-2,2,6,6-tetramethyl-piperidin-1-oxyl,
3-Carbamoyl-2,2,5,5-tetramethyl-3-pyrrolin-1-oxyl,
4-Phenyl-2,2,5,5-tetramethyl-3-imidazolin-3-oxid-1-oxyl,
4-Carbamoyl-2,2,5,5-tetramethyl-3-imidazolin-3-oxid-1-oxyl,
4-Phenacyliden-2,2,5,5-tetramethyl-imidazolidin-1-oxyl.

Als Mediatoren insbesondere bevorzugt sind
2,2,6,6-Tetramethyl-piperidin-1-oxyl (TEMPO), und
4-Hydroxy-2,2,6,6-tetramethyl-piperidin-1-oxyl.

Die Vermeidung eines schädlichen Mediatoreinflusses aus das Gesamtsystem kann auch dadurch erreicht werden, daß eine Hydrolase der Enzymklasse 3.2.1 verwendet wird, welche gegenüber einer Modifikation durch aktivierten Mediator keine für die beschriebene Verwendung nachteilige Änderung ihrer enzymatischen Eigenschaften zeigt (z.B. eine Mediator-resistente Xylanase oder Zellulase).

Das erfindungsgemäße Mehrkomponentensystem umfaßt mindestens ein enzymatisch wirksames Additiv, ausgewählt aus der Gruppe der Hydrolasen der Enzymklasse 3.2.1.

Bevorzugt handelt es sich bei der Hydrolase der Enzymklasse 3.2.1 um Hemizellulasen, z.B. Xylanasen, Mannanasen oder Zellulasen.

Inbesondere bevorzugt handelt es sich bei dem enzymatisch wirksamen Additiv um eine Endo 1,4-β-Xylanase (Enzymklasse 3.2.1.8) und/oder eine Endo 1,4-β-Glucanase (Enzymklasse 3.2.1.4).

Die Hydrolasen der Enzymklasse 3.2.1 sind käuflich erhältlich oder lassen sich nach Standardverfahren gewinnen. Die Hydrolasen der Enzymklasse 3.2.1 können entweder aus den natürlichen Organismen gewonnen werden oder mittels bekannter rekombinanter DNA-Technologie in geeigneten Expressionssystemen produziert und entsprechend dem Stand der Technik aus dem Kulturmedium isoliert werden. Als Organismen zur Produktion dieser Enzyme kommen beispielsweise Pflanzen, tierische Zellen, Bakterien und Pilze in Betracht. Grundsätzlich können sowohl natürlich vorkommende als auch gentechnisch veränderte Organismen Enzymproduzenten sein. Ebenso sind Teile von einzelligen oder mehrzelligen Organismen als Enzymproduzenten denkbar, vor allem Zellkulturen.

Die Hydrolasen der Enzymklasse 3.2.1 können auch durch bekannte molekularbiologische Verfahren in einer Weise verändert worden sein, daß sie für die beschriebene Anwendung geeignet sind, z.B. dadurch, daß sie gegenüber dem aktivierten Mediator oder einer proteolytischen Aktivität unempfindlich gemacht wurden.

Die Erfindung betrifft ferner Verfahren zur Delignifizierung von ligninhaltigen Materialien, die dadurch gekennnzeichnet sind, daß mindestens eine Oxidoreduktase und mindestens ein für die Oxidoreduktase geeignetes Oxidationsmittel und mindestens ein Mediator, der ein Enzym, ausgewählt aus der Gruppe der Oxidoreduktasen und Hydrolasen der Enzymklasse 3.2.1., nicht inaktiviert und mindestens eine Endohydrolase gleichzeitig oder in beliebiger Reihenfolge mit einer wässrigen Suspension des ligninhaltigen Materials mischt.

Überraschenderweise gelingt es im erfindungsgemäßen Verfahren, das oxidative Delignifiziersystem aus Laccase und einer Mediatorsubstanz mit einem hydrolytischen Enzym, wie z.B. der Xylanase und/oder der Zellulase in einem einzigen Verfahrensschritt zu kombinieren.

Dies ist um so überraschender, als bekannt ist, daß bei Verwendung von HBT als Mediator die Laccase selbst in ihrer Aktivität beeinträchtigt wird (Amann, 1997) und gleichzeitig anwesende Enzyme irreversibel geschädigt werden und damit stark verringerte Aktivität zeigen (Oksanen, 1997).

Das erfindungsgemäße Verfahren hat folgende Vorteile:
- Es verbessert die Bleichbarkeit von Zellstoff
- es läßt sich unproblematisch mit etablierten chemischen Verfahrensstufen kombinieren.

Beispielsweise auch in Kombination mit üblichen Verfahrensschritten aus der Zellstoffherstellung, z.B. einer alkalischen Extraktion, läßt sich Lignin selektiv aus dem eingesetzten Material entfernen.

Für das erfindungsgemäße Verfahren wird beispielsweise ligninhaltiges Material pflanzlichen Ursprungs verwendet, vorzugsweise solches, welches mit den für die Holzstoff- oder Zellstoffherstellung üblichen Verfahren aufgeschlossen wurde.

Das eingesetzte Material kann ungebleichter Zellstoff oder Material sein, welches bereits mit chemischen Delignifizier- und Bleichverfahren behandelt worden ist.

Im erfindungsgemäßen Verfahren werden vorzugsweise 0,1 - 100 IU Oxidoreduktase pro g ligninhaltiges Material (Trockengewicht), besonders bevorzugt 0,5 - 20 IU pro g ligninhaltiges Material eingesetzt.

Die Konzentration des Oxidationsmittels liegt vorzugsweise im Bereich von 0,001 bis 50 mmol/l. Wenn als Oxidationsmittel Wasserstoffperoxid eingesetzt wird, so vorzugsweise im Bereich von 0,001 - 25 mmol/l.

Der Mediator wird vorzugsweise eingesetzt in Mengen von
1 - 500 mmol/kg Zellstoff, besonders bevorzugt in Mengen von
5 - 300 mmol/kg Zellstoff, insbesondere in Mengen von
10 - 200 mmol/kg Zellstoff (atro - absolut trocken).

Im erfindungsgemäßen Verfahren werden vorzugsweise Zellulasen und/oder Hemizellulasen als enzymatische Additive verwendet. Beispielsweise können Zellulase, Xylanasen oder Mannanasen in reiner Form oder in Form von Mischungen verwendet werden. Xylanasen werden bereits in der Bearbeitung von Zellstoffen verwendet und von mehreren Herstellern , z.B. Novo-Nordisk (Pulpzyme®), Clariant (Cartazyme®) angeboten. Zellulasen sind ebenfalls käuflich erhältlich.

Je nach den Bedingungen, welche in dem erfindungsgemäßen Verfahren für die enzymatische Delignifizierung eingestellt werden, können entsprechend gut geeignete Varianten (pH-Optimum, Temperaturstabilität) dieser Enzyme verwendet werden.

In dem erfindungsgemäßen Verfahren wird die Hydrolase der Enzymklasse 3.2.1 in Dosierungen von 0,01 - 1000 IU Enzym/g Zellstoff verwendet, bevorzugt in Dosierungen von 1 - 500 IU Enzym/g, besonders bevorzugt in Dosierungen von 5 - 100 IU/g Zellstoff (atro - absolut trocken).

Hemizellulasen werden vorzugsweise in Dosierungen von 0,1 - 1000 IU Enzym/g Zellstoff verwendet, bevorzugt in Dosierungen von 1 - 500 IU Enzym/g, besonders bevorzugt in Dosierungen von 5 - 100 IU/g Zellstoff (atro - absolut trocken).

Zellulasen werden vorzugsweise in Dosierungen von 0,01 - 500 IU Enzym/g Zellstoff verwendet, bevorzugt 0,05 - 100 IU Enzym/g Zellstoff, besonders bevorzugt 0,1 - 10 IU Enzym/g Zellstoff eingesetzt.

Üblicherweise wird das erfindungsgemäße Verfahren bei Temperaturen zwischen 40 und 90°C, bevorzugt zwischen 45 und 70°C, besonders bevorzugt zwischen 45 und 65°C durchgeführt.

Je nach Temperaturstabilität der verwendeten Enzyme können auch davon unterschiedliche optimale Reaktionsbedingungen eingestellt werden. Generell lassen sich im pH-Bereich von pH 3 - pH 10 gute Delignifizierergebnisse erzielen, besonderes bevorzugt ist der pH-Bereich von pH 4,5 - 7.

Bei Verwendung von Laccasen ist die ausreichende Verfügbarkeit von gelöstem Sauerstoff eine notwendige Bedingung. Je nach gewählter Temperatur kann die ausreichende Sauerstoffkonzentration durch Anlegen eines genügend hohen Drucks an das System gewährleistet werden. Der notwendige Sauerstoffpartialdruck kann entweder bereits durch den hydrostatischen Druck gewährleistet sein oder durch Anlegen eines geeigneten Drucks mittels eines geeigneten, sauerstoffhaltigen Gasgemisches, wie z.B. Luft oder auch reines Sauerstoffgas, erreicht werden.

Der übliche Druckbereich liegt im Bereich eines Sauerstoff-Partialdrucks (pO₂) von 0,1 - 20 bar, bevorzugt im Druckbereich von 0,3 - 10 bar.

Je nach Wahl des Mediators, der Enzymdosierungen und der Reaktionsbedingungen kann das Verfahren unterschiedlich schnell ablaufen. Die übliche Reaktionsdauer beträgt zwischen 30 min. und 2 Stunden.

Die Zellstoffkonzentration ( Stoffdichte) des erfindungsgemäßen Verfahrens liegt üblicherweise im Bereich von 1 - 25%, bevorzugt im Bereich 6 - 20%, besonders bevorzugt im Bereich 10 -15%.

In den nachfolgenden Beispielen wird die Erfindung weiter beschrieben.

Folgende Komponenten werden in den Beispielen eingesetzt:
Mediatoren:
   Mediatoren wurden von den Firmen Aldrich, Merck oder Janssen bezogen oder nach üblichen und allgemein bekannten Verfahren hergestellt. Einige Mediatoren werden im Text wie folgt mit Abkürzungen bezeichnet: 1-OH-Benzotriazol (HBT), Violursäure (Vio), N-OH-Acetanilid (NHA).
Enzyme:
Laccase:
   Fermentativ gewonnene Laccase aus Trametes versicolor wurde verwendet.
Aktivitätsbestimmung:
   Laccaseaktivität wird über die Oxidation von 2,2'-Azino-bis-(3-ethylbenzothiazoline-6-sulfonate), ABTS, Boehringer Mannheim, unter aeroben Bedingungen bestimmt. Die grüne Farbe, welche durch die enzymatische Reaktion entsteht, wird photometrisch bei 420 nm gemessen. Die Reaktionstemperatur beträgt 25°C, der pH 4,5. Eine Laccase-Unit ist die Menge an Enzym, welche die Konversion von 1 mol ABTS/min unter den angegebenen Bedingungen katalysiert. Zur Berechnung des Umsatzes wird ein Extinktionskoeffizient von 36000 M⁻¹cm⁻¹ verwendet.
Zellulase:
   Verwendet wurden kommerziell erhältliche Zellulasen der Firmen Fluka (Trichoderma viride) , Sigma (Aspergillus niger, Trichoderma viride, Penicillium funiculosum), Röhm (Rohalase 7069). Die Dosierung erfolgte entsprechend den Aktivitätsangaben der Hersteller.
Xylanase:
   Verwendet wurden kommerziell erhältliche Xylanasen der Firmen Fluka (Trichoderma viride) oder Clariant. Die Dosierung erfolgte entsprechend den Aktivitätsangaben der Hersteller.
Zellstoffe:
   Verwendet wurden Hardwood (HW) und Softwood (SW) Kraft Zellstoffe.
Softwood : Kappa 14,5 (nach Extraktion)
Hardwood : Kappa 12 (nach Extraktion)

Die Bewertung der Delignifizierung erfolgte durch Bestimmung des Kappa-Werts (nach TAPPI Methode T236) der behandelten Zellstoffprobe nach alkalischer Extraktion. Die alkalische Extraktion (E-Stufe) erfolgte bei 2% Stoffdichte, 60°C mit 80g NaOH/kg Zellstoff und einer Dauer von 60 min.

### Beispiel 1

### Vergleich der Delignifizierung eines SW-Zellstoffes mit einem Laccase-Mediator System (LMS®) bei gleichzeitiger Anwesenheit/Abwesenheit von Xylanase

In einem 500 ml Laborautoklaven wurde nachfolgend tabellarisch beschriebener Ansatz zwei Stunden lang bei 45°C (Wasserbad) unter Anlegen eines Sauerstoffpartialdrucks von 10 bar inkubiert (L-Stufe).

### L-Stufe

### 5 g Softwood-Zellstoff (atro)

Stoffdichte: 10%
Laccase: 5 IU/ g Zellstoff
Mediator: N-OH-Acetanilid: 9 mg/g Zellstoff
pH- Wert: 4,5
Gesamtflüssigkeitsvolumen: 50 ml

Um den positiven Effekt eines enzymatischen Additivs, wie z.B. der Xylanase, zu bestimmen, wurde der beschriebenen L-Stufe als weiterer Zusatz Xylanase von Trichoderma viride (Fluka) zugegeben (XL-Stufe), so daß der Ansatz wie folgt zusammengesetzt war:

### (XL)-Stufe

### 5 g Softwood-Zellstoff (atro)

Stoffdichte: 10%
Laccase: 5 IU/g Zellstoff
Xylanase: 50 IU/g Zellstoff
Mediator: N-OH-Acetanilid: 9 mg/g Zellstoff
pH- Wert: 4,5
Gesamtflüssigkeitsvolumen: 50 ml

Beide Ansätze wurden anschließend 60 min. lang alkalisch extrahiert(E-Stufe).

### E-Stufe

### 2 % Stoffdichte

Temperatur: 60°C
Alkali: 80g NaOH/kg Zellstoff

Aus der Bestimmung der Kappa-Werte wurde der Grad der Delignifizierung bestimmt. Der Kontrollwert gibt den Kappa-Wert des Ausgangszellstoffs nach alkalischer Extraktion an.

**Tabelle 1**

| **Versuchsauswertung** | | |
|---|---|---|
| Behandlung | Kappa-Wert | Delignifizierung |
| Kontrolle E | 14,5 | 0 |
| LE (St. d. T) | 10,9 | 25% |
| (XL) E | 9,8 | 32,4% |
| (St. d. T = Versuch gemäß Stand der Technik/ Vergleichsversuch) | | |

Der Zusatz von Xylanase steigert die Delignifizierung um ca. 30%.

### Beispiel 2

### Vergleich der Delignifizierung eines HW-Zellstoffs mit einem Laccase-Mediator System (LMS^{®}) bei gleichzeitiger Anwesenheit/Abwesenheit von Xylanase

Das Beispiel entspricht dem Beispiel 1, mit dem Unterschied, daß anstelle von Softwood- ein Hardwood-Zellstoff verwendet wurde. In einem 500 ml-Laborautoklaven wurde nachfolgend tabellarisch beschriebener Ansatz zwei Stunden lang bei 45°C (Wasserbad) unter Anlegen eines Sauerstoffpartialdrucks von 10 bar inkubiert ( L-Stufe).

### L-Stufe

### 5 g Hardwood -Zellstoff (atro)

Stoffdichte: 10%
Laccase: 5 IU/g Zellstoff
Mediator: N-OH-Acetanilid: 9 mg/g Zellstoff
pH- Wert: 4,5
Gesamtflüssigkeitsvolumen: 50 ml

Um den positiven Effekt eines enzymatischen Additivs, wie z.B. der Xylanase, zu bestimmen, wurde der beschriebenen L-Stufe als weiterer Zusatz Xylanase von Trichoderma viride (Fluka) zugegeben (XL-Stufe), so daß der Ansatz wie folgt zusammengesetzt war:

### (XL) -Stufe

### 5 g Hardwood-Zellstoff (atro)

Stoffdichte: 10%
Laccase: 5 IU/g Zellstoff
Xylanase: 50 IU/g Zellstoff
Mediator: N-OH-Acetanilid: 9 mg/g Zellstoff
pH- Wert: 4,5
Gesamtflüssigkeitsvolumen: 50 ml

Die Ansätze wurden anschließend 60 min. lang alkalisch extrahiert (E-Stufe).

### E-Stufe

### 2% Stoffdichte

Temperatur: 60°C
Alkali: 80g NaOH/. kg Zellstoff

Aus der Bestimmung der Kappa-Werte wurde der Grad der Delignifizierung bestimmt. Der Kontrollwert gibt den Kappa-Wert des Ausgangszellstoffs nach alkalischer Extraktion an.

**Tabelle 2**

| **Versuchsauswertung** | | |
|---|---|---|
| Behandlung | Kappa-Wert | Delignifizierung |
| Kontrolle E | 12 | 0 |
| LE (St. d. T) | 10,3 | 14% |
| (XL) E | 8,7 | 27,5% |
| Der Zusatz von Xylanase steigert die Delignifizierung um ca. 96 %. | | |

### Beispiel 3

### Einfluß verschiedener Xylanasen auf die Delignifizierung eines Zellstoffs mit einem Laccase-Mediator System (LMS^{®})

Um die Auswirkungen des Zusatzes von unterschiedlichen Xylanasen zu untersuchen, wurde die Delignifizierungswirkung entsprechend der in Beispiel 1 beschriebenen (XL)-Stufe bei Verwendung unterschiedlicher Xylanasen untersucht. Als Xylanasen wurden verwendet:
Fa. Clariant : Cartazym HS 10, liquid
Fa. Fluka: Trichoderma viride Xylanase

L und (XL)-Stufe wurden entsprechend Beispiel 1 durchgeführt, jedoch wurden die Ansätze bei pH 6,0 gefahren. Die Xylanasedosierung betrug jeweils 12 IU/g Zellstoff, Laccase wurde mit 15 IU/g Zellstoff dosiert.
Die Ansätze wurden anschließend 60 min. lang alkalisch extrahiert (E-Stufe, Beispiel 1) . Aus der Bestimmung der Kappa-Werte wurde der Grad der Delignifizierung bestimmt.

**Tabelle 3**

| **Ergebnisse** | | |
|---|---|---|
| verwendete Xylanase | Kappa-Wert | Delig. |
| keine (St. d. T.) | 11,3 | 21,8 |
| Fa. Clariant "Cartazym" | 11 | 24,1 |
| Fa. Fluka "Trichoderma viride" | 9,6 | 33,6 |

Um eine optimale Delignifizierung zu erzielen, müssen die jeweils besten Kombinationen von Zellstoff, LMS und Xylanase, ermittelt werden. Verschiedene Xylanasen führen, wie das Beispiel zeigt, zu unterschiedlich guten Delignifizierungen. Alle Ergebnisse sind jedoch besser als ohne Xylanase.

### Beispiel 4

### Mediatorabhängigkeit der Delignifizierung mit einem Laccase-Mediator-System (LMS^{®}) bei gleichzeitiger Anwesenheit von Xylanase

Um den Einfluß des Mediators auf das System zu bestimmen, wurde die Delignifizierungswirkung der in Beispiel 1 beschriebenen (XL)-Stufe bei Verwendung unterschiedlicher Mediatoren untersucht.
Als Mediatoren wurden verwendet (Abkürzungen in Klammern):
1-OH-Benzotriazol (HBT), Violursäure (Vio), N-OH-Acetanilid (NHA), N-OH-Phthalimid (HPI),
1-Nitroso-2-naphthol-3,6-disulfonsäure Dinatriumsalz Hydrat
(1-NNS), 2-Nitroso-1-naphthol-4-sulfonsäure Tetrahydrat (2-NNS).

Die Ansätze wurden wie in Beispiel 1 durchführt. Im einzelnen enthielten sie:

### (XL)-Stufe (Mediatorabhängigkeit)

### 5 g Softwood-Zellstoff (atro)

Stoffdichte: 10%
Laccase: 15 IU/g Zellstoff
Xylanase (Trichoderma): 25 IU/g Zellstoff
Mediatoren: 75 mmol/kg Zellstoff
pH- Wert: 6,0
Gesamtflüssigkeitsvolumen: 50 ml

Die Ansätze wurden anschließend 60 min. lang alkalisch extrahiert (E-Stufe), wie in Beispiel 1 beschrieben.
Aus der Bestimmung der Kappa-Werte wurde der Grad der Delignifizierung (Delig.) bestimmt. Der Kontrollwert gibt den Kappa-Wert des Ausgangszellstoffs nach alkalischer Extraktion an.

**Tabelle 4**

| **Versuchsauswertung** | | | | | |
|---|---|---|---|---|---|
| Mediator | Kappa-Wert | Delig. (%) | Kappa-Wert | Delig. (%) | Steigerung der Delignifizierung (ohne Xyl/mit Xyl) |
| | ohne Xyl | ohne Xyl | mit Xyl | mit Xyl | |
| ohne (Ktrl.) | 14,5 | 0 | 14,5 | 0 | 0% |
| HBT (St.d.T) | 12,2 | 15,9 | 11,6 | 20 | 5,0% |
| Vio | 12,5 | 13,8 | 10,5 | 27,6 | 19,0% |
| NHA | 10,6 | 26,9 | 9 | 37,9 | 17,8% |
| 1-NNS | 10,8 | 25,5 | 8,8 | 39,3 | 22,7% |
| 2-NNS | 10,5 | 27,6 | 8,8 | 39,3 | 19,3% |
| HPI | 13,3 | 8,3 | 11,5 | 20,6 | 15,6% |
| Xyl : Xylanase | | | | | |

Der Xylanase-Effekt hängt stark vom verwendeten Mediator ab. Während sich die Delignifizierung bei Verwendung von 1-OH-Benzotriazol (HBT) fast nicht steigern läßt (+5%), können mit anderen Mediatoren signifikante Steigerungen beobachtet werden (Bsp. NHA +17,8%).

### Beispiel 5

### pH-Abhängigkeit der Delignifizierung eines Zellstoffs mit einem Laccase-Mediator-System (LMS^{®}) bei gleichzeitiger Anwesenheit von Xylanase

Um den Einfluß des pH-Werts auf das System zu bestimmen, wurde die Delignifizierungswirkung der in Beispiel 1 beschriebenen (XL)-Stufe bei unterschiedlichen pH-Werten untersucht. Als Vergleich dienten entsprechende Ansätze ohne Xylanase (L-Stufe). Die Ansätze wurden wie in Beispiel 1 durchführt. Im einzelnen enthielten sie:

### (XL)-Stufe - (pH-Ahhängigkeit)

### 5 g Softwood-Zellstoff (atro) - Kappa 14,5

Stoffdichte: 10%
Laccase: 15 IU/g Zellstoff
Xylanase (Trichoderma viride): 25 IU/g Zellstoff
Mediator: N-OH-Acetanilid: 9 mg/g Zellstoff
pH- Werte: 4/5/6/7 ( mit McIllvaine Puffer)
Gesamtflüssigkeitsvolumen: 50 ml

Beide Ansätze wurden anschließend 60 min. lang alkalisch extrahiert (E-Stufe).
Aus der Bestimmung der Kappa-Werte wurde der Grad der Delignifizierung bestimmt.

**Tabelle 5**

| **Versuchsauswertung** | | | | |
|---|---|---|---|---|
| pH-Wert | Kappa-Wert (K) | Delignifizierung | Kappa-Wert (K) | Delignifizierung |
| | ohne Xyl | ohne Xyl | mit Xyl | mit Xyl |
| 4 | 11,3 | 22% | 10 | 31% |
| 5 | 11 | 24% | 9,3 | 36% |
| 6 | 10 | 31% | 8,5 | 41% |
| 7 | 12,4 | 14,5% | 11,2 | 23% |

Durch den Zusatz von Xylanase zum Laccase-Mediator System kann eine drastische Verbesserung der Performance erzielt werden. Der Effekt zeigt eine deutliche pH-Abhängigkeit. Bei allen pH-Werten ist jedoch durch den Zusatz von Xylanase eine Verbesserung zu erreichen.

### Beispiel 6

### Abhängigkeit der Delignifizierung eines Zellstoffs mit einem Laccase-Mediator-System (LMS^{®}) bei gleichzeitiger Anwesenheit unterschiedlicher Menge Xylanase (Dosisabhängigkeit)

Um den Einfluß der Xylanase-Dosierung auf das System zu bestimmen, wurde die Delignifizierungswirkung der in Beispiel 1 beschriebenen (XL)-Stufe bei unterschiedlichen Xylanase-Dosierungen untersucht. Als Vergleich diente ein entsprechender Ansatz ohne Xylanase (L-Stufe). Die Ansätze wurden wie in Beispiel 1 durchführt. Im einzelnen enthielten sie:

### L-Stufe (Referenzwert ohne Xylanase)

### 5 g Softwood-Zellstoff (atro)

Stoffdichte: 10%
Laccase: 5 IU/g Zellstoff
Mediator: N-OH-Acetanilid: 9 mg/g Zellstoff
pH-Wert: 4,5
Gesamtflüssigkeitsvolumen: 50 ml

### (XL)-Stufe - (versch. Xylanase-Mengen)

### 5 g Softwood-Zellstoff (atro) - Kappa 14,5

Stoffdichte: 10%
Laccase: 5 IU/g Zellstoff
Xylanase (Trichoderma viride): 12,5/25/37,5/50/100/200 IU/g Zellstoff
Mediator: N-OH-Acetanilid: 11 mg/g Zellstoff
pH- Wert: 4,5
Gesamtflüssigkeitsvolumen: 50 ml

Nach der Inkubation wurden die Ansätze 60 min. lang alkalisch extrahiert (E-Stufe).
Aus der Bestimmung der Kappa-Werte wurde der Grad der Delignifizierung bestimmt.

**Tabelle 6**

| **Versuchsauswertung** | | | |
|---|---|---|---|
| Xylanase Dosierung (IU/g) | Kappa-Wert | Delignifizierung | relative Steigerung der Delignifizierung |
| 0 | 10 | 31% | - |
| 6,3 | 9,3 | 36% | 16% |
| 12,5 | 8,4 | 42% | 35% |
| 25 | 8,5 | 41% | 32% |
| 37,5 | 8,9 | 39% | 26% |
| 50 | 10,1 | 30% | - 3% |
| 100 | 10 | 31% | 0% |
| 200 | 10,2 | 30% | - 3% |

Die Verbesserung der Effektivität des Laccase-Mediator-systems ist abhängig von der zugegebenen Dosis an Xylanase.

### Beispiel 7

### Delignifizierung eines SW - Zellstoffs mit einem Laccase-Mediator System (LMS^{®}) welches als Additiv Zellulase enthält.

In einem 500 ml Laborautoklaven wurde nachfolgend tabellarisch beschriebener Referenzansatz 2 Stunden lang bei 45°C (Wasserbad) unter Anlegen eines Sauerstoffpartialdrucks von 10 bar inkubiert (L-Stufe).

### L-Stufe (Vergleichsversuch)

### 5 g Softwood-Zellstoff (atro)

Stoffdichte: 10 %
Laccase: 15 IU/ g Zellstoff
Mediator: N-OH-Acetanilid: 10 mg / g Zellstoff
pH- Wert: 4,5
Gesamtflüssigkeitsvolumen: 50 ml

Um den positiven Effekt eines enzymatischen Additivs, wie z.B. der Zellulase, zu bestimmen, wurde einem der L-Stufe entsprechenden Ansatz als weiterer Zusatz Zellulase von Trichoderma viride (Sigma) zugegeben (CL-Stufe), so daß der Ansatz wie folgt zusammengesetzt war:

### (CL)-Stufe

### 5 g Softwood-Zellstoff (atro)

Stoffdichte: 10 %
Laccase: 15 IU/ g Zellstoff
Zellulase: 10 U / g Zellstoff
Mediator: N-OH-Acetanilid: 10 mg / g Zellstoff
pH- Wert: 4,5
Gesamtflüssigkeitsvolumen: 50 ml

Beide Ansätze wurden anschließend 60 min lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte beider Ansätze wurde der Grad der Delignifizierung bestimmt. Der Kontrollwert gibt den Kappa-Wert des Ausgangszellstoffs nach alkalischer Extraktion an.

**Tabelle 7**

| **Versuchsauswertung** | | |
|---|---|---|
| Behandlung | Kappa-Wert | Delignifizierung |
| Kontrolle E | 16,6 | 0 |
| (CL) E | 11 | 33,7 % |

Der Zusatz von Zellulase steigert die Delignifizierung eines Softwood Zellstoffs gegenüber einer Zellulase freien Referenz um 43 %.

### Beispiel 8

### Delignifizierung eines HW - Zellstoffs mit einem Laccase-Mediator System (LMS^{®}) welches als Additiv Zellulase enthält.

In einem 500 ml Laborautoklaven wurde nachfolgend tabellarisch beschriebener Referenzansatz 2 Stunden lang bei 45°C (Wasserbad) unter Anlegen eines Sauerstoffpartialdrucks von 10 bar inkubiert (L-Stufe).

### L-Stufe (Vergleichsversuch)

### 5 g Hardwood-Zellstoff (atro)

Stoffdichte: 10 %
Laccase: 15 IU/ g Zellstoff
Mediator: N-OH-Acetanilid: 10 mg / g Zellstoff
pH- Wert: 4,5
Gesamtflüssigkeitsvolumen: 50 ml

Um den positiven Effekt eines enzymatischen Additivs , wie z.B. der Zellulase, zu bestimmen, wurde einem der L-Stufe entsprechenden Ansatz als weiterer Zusatz Zellulase von Trichoderma viride (Sigma) zugegeben (CL-Stufe), so daß der Ansatz wie folgt zusammengesetzt war:

### (CL)-Stufe

### 5 g Hardwood-Zellstoff (atro)

Stoffdichte: 10 %
Laccase: 15 IU/ g Zellstoff
Zellulase: 10 U / g Zellstoff
Mediator: N-OH-Acetanilid: 10 mg / g Zellstoff
pH- Wert: 4,5
Gesamtflüssigkeitsvolumen: 50 ml

Beide Ansätze wurden anschließend 60 min. lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte beider Ansätze wurde der Grad der Delignifizierung bestimmt. Der Kontrollwert gibt den Kappa-Wert des Ausgangszellstoffs nach alkalischer Extraktion an.

**Tabelle 8**

| **Versuchsauswertung** | | |
|---|---|---|
| Behandlung | Kappa-Wert | Delignifizierung |
| Kontrolle E | 12 | 0 % |
| LE | 10,5 | 12,4 % |
| (CL) E | 8,9 | 25,8 % |

Der Zusatz von Zellulase steigert die Delignifizierung eines Hardwood Zellstoffs gegenüber einer zellulasefreien Referenz um 108 %.

### Beispiel 9

### Beeinflussung des Zellulaseaktivität durch das Laccase-Mediator System (LMS^{®}) bei Verwendung unterschiedlicher Mediatoren

Ein Effekt auf die Delignifizierbarkeit von Zellstoff kann nur dann beobachtet werden, wenn die Zellulase in Gegenwart des Laccase-Mediator Systems enzymatisch aktiv ist. Die Aktivität von Zellulasen wurde daher in einem zellstofffreien System, welches Laccase und Mediator enthielt, gemessen. Untersucht wurde Zellulase aus Aspergillus niger und Trichoderma viride. Als Mediatoren wurden 1-OH-Benzotriazol (HBT), Violursäure (Vio) und N-OH-Acetanilid (NHA) verwendet. Die Messung des Zellulaseaktivität erfolgte in einem optischen Test mit Dinitrosalicylsäure (DNSA) als Oxidationsmittel wie nachstehend beschrieben.

### Ansätze

Die Ansätze enthielten in einem Gesamtvolumen von 10 ml 20 mg Zellulase, 0,8 mg Laccase und 75 µmol Mediator bei einem pH-Wert von 6,2. Dem Ansatz wurden nach unterschiedlichen Zeiten je 50 µl entnommen und die Zellulaseaktivität bestimmt.

### Zellulasetest

Prinzip: Bestimmung der Menge an reduzierendem Zucker, welche durch die enzymatische Hydrolyse aus Carboxymethylzellulose (CMC) freigesetzt wird. Eine nachgeschaltete Redoxreaktion zwischen zugesetztem DNSA und den enzymatisch gebildeten reduzierenden Zukkerenden führt zu einer Färbung der Lösung, welche photometrisch bei 595 nm quantifiziert werden kann.

Für den Enzymtest werden 325 µl Testpuffer (0,1 mol/l Citrat/Phosphat pH 6,2 mit 0,4 mol/l NaCl), 125 µl CMC-Lösung (2% in Testpuffer; Na-Salz) und 50 µl der zu testenden zellulasehaltigen Lösung zusammenpipettiert und bei 45°C unterschiedlich lang inkubiert. Nach Ablauf der Inkubation werden jedem Ansatz 750 µl DNSA-Lösung ( 1g 3,5-Dinitrosalicylsäure, 0,2 g Phenol, 0,05g Na₂SO₃ und 20 g NaK-Tartrat in 100 ml 1%iger NaOH lösen) zugegeben und die Mischung anschließend 20 min bei 95°C erhitzt. Die Lösung wird auf Eis abgekühlt, eventuell ausfallendes Material abzentrifugiert (2 min, 14000 rpm, Eppendorf Zentrifuge) und im Überstand die Extinktion bei 595 nm bestimmt. Durch Vergleich mit einer Eichkurve (Glucose) kann die Bildung reduzierender Zucker quantifiziert werden. Als 1 Unit (1U) wird dabei diejenige Enzymaktivität bezeichnet, welch in einer Minute 1 µmol Glucose unter den angegebenen Bedingungen aus CMC-Zellulose freisetzt.

**Tabelle 9**

| | | | |
|---|---|---|---|
| Zeitabhängigkeit der Zellulaseaktivität von Aspergillus niger in Gegenwart des Laccase-Mediator Systems bei Verwendung verschiedener Mediatoren (HBT, NHA, Vio). Die Zahlen geben die relative Zellulaseaktivität in Prozent des Ausgangswerts an (rel %). | | | |

| Zeit (min) | NHA | HBT | Vio |
|---|---|---|---|
| 0 | 100 | 100 | 100 |
| 30 | 81 | 49 | 43 |
| 60 | 65 | 38 | 39 |
| 120 | 47 | 40 | 40 |
| 170 | 45 | 43 | 38 |
| 1.300 | 43 | 42 | 41 |

**Tabelle 10**

| | | | |
|---|---|---|---|
| Zeitabhängigkeit der Zellulaseaktivität von Trichoderma viride in Gegenwart des Laccase-Mediator Systems bei Verwendung verschiedener Mediatoren (HBT, NHA, Vio). Die Zahlen geben die relative Zellulaseaktivität in Prozent des Ausgangswerts an (rel %). | | | |

| Zeit (min) | NHA | HBT | Vio |
|---|---|---|---|
| 0 | 100 | 100 | 100 |
| 60 | 95 | 96 | 95 |
| 120 | 92 | 82 | 91 |
| 170 | 77 | 75 | 64 |
| 1.300 | 70 | 33 | 18 |

Die enzymatische Aktivität der Zellulasen aus A.niger und T.viride nimmt bei gleichzeitiger Inkubation im Laccase-Mediator System ab. Die geringsten Einbußen findet man mit NHA als Mediator. Eine Abhängigkeit vom verwendeten Enzym ist ebenfalls zu beobachten.

### Beispiel 10

### Delignifizierung eines SW - Zellstoffs mit einem Laccase-Mediator System (LMS^{®}) welches als Additiv Zellulase unterschiedlicher Herkunft enthält

In einem 500 ml Laborautoklaven wurde nachfolgend tabellarisch beschriebener Referenzansatz 2 Stunden lang bei 45°C (Wasserbad) unter Anlegen eines Sauerstoffpartialdrucks von 10 bar inkubiert ( L-Stufe).

### L-Stufe (Vergleichsversuch)

### 5 g Softwood-Zellstoff (atro)

Stoffdichte: 10 %
Laccase: 15 IU/ g Zellstoff
Mediator: N-OH-Acetanilid: 10 mg / g Zellstoff
pH- Wert: je nach Zellulase pH 5 - 6 (Tabelle)
Gesamtflüssigkeitsvolumen: 50 ml

Um den positiven Effekt eines enzymatischen Additivs , wie z.B. der Zellulase, zu bestimmen, wurde einem der L-Stufe entsprechenden Ansatz als weiterer Zusatz Zellulose unterschiedlicher Hersteller zugegeben (CL-Stufe), so daß der Ansatz wie folgt zusammengesetzt war:

### (CL)-Stufe

### 5 g Softwood-Zellstoff (atro)

Stoffdichte: 10 %
Laccase: 15 IU/ g Zellstoff
Zellulase (versch. Herkunft): 10 U / g Zellstoff
Mediator: N-OH-Acetanilid: 10 mg / g Zellstoff
pH- Wert: je nach Zellulase pH 5 - 6 (Tabelle 11)
Gesamtflüssigkeitsvolumen: 50 ml

Beide Ansätze wurden anschließend 60 min lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte beider Ansätze wurde der Grad der Delignifizierung bestimmt. Der Kontrollwert gibt den Kappa-Wert des Ausgangszellstoffs nach alkalischer Extraktion an.

**Tabelle 11**

| **Versuchsauswertung** | | | | |
|---|---|---|---|---|
| Verbesserung der Delignifizierung mit dem Laccase-Mediator System bei gleichzeitiger Anwesenheit von Zellulasen unterschiedlicher Herkunft. | | | | |

| Zellulase | Firma | pH | Delignifizierung (%) | Steigerung gegenüber Kontrolle |
|---|---|---|---|---|
| Keine (Kontrolle) | - | pH 5 | 23,5 | 0 |
| Aspergillus niger | Sigma | pH 5 | 26,0 | 10 |
| Trichoderma viride | Sigma | pH 6 | 33,7 | 43 |
| Penicillium funiculosum | Sigma | pH 6 | 31,3 | 33 |
| Rohalase 7069 | Röhm | pH 6 | 32,5 | 38 |

Mit allen verwendeten Zellulase kann die Delignifizierleistung des Laccase-Mediator-Systems verbessert werden.

### Beispiel 11

### Einfluß der Zellulasedosis auf die Delignifizierung mittels eines Laccase-Mediator Systems (LMS^{®}) welches als Additiv Zellulase enthält.

In einem 500 ml Laborautoklaven wurde nachfolgend tabellarisch beschriebener Referenzansatz 2 Stunden lang bei 45°C (Wasserbad) unter Anlegen eines Sauerstoffpartialdrucks von 10 bar inkubiert ( L-Stufe).

### L-Stufe (Vergleichsversuch)

### 5 g Softwood-Zellstoff (atro)

Stoffdichte: 10 %
Laccase: 15 IU/ g Zellstoff
Mediator: N-OH-Acetanilid: 10 mg / g Zellstoff
pH- Wert: pH 6
Gesamtflüssigkeitsvolumen: 50 ml

Um den positiven Effekt des enzymatischen Additivs (Zellulase) zu bestimmen, wurde einem der L-Stufe entsprechenden Ansatz als weiterer Zusatz verschiedene Mengen an Zellulase zugegeben (CL-Stufe), so daß der Ansatz wie folgt zusammengesetzt war:

### (CL)-Stufe

### 5 g Softwood-Zellstoff (atro)

Stoffdichte: 10 %
Laccase: 15 IU/ g Zellstoff
Zellulase (Trichoderma viride - Sigma) 0 - 30 U / g Zellstoff
Mediator: N-OH-Acetanilid: 10 mg / g Zellstoff
pH-Wert: 6
Gesamtflüssigkeitsvolumen: 50 ml

Beide Aßsätze wurden anschließend 60 min lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte beider Ansätze wurde der Grad der Delignifizierung bestimmt. Der Kontrollwert gibt den Kappa-Wert des Ausgangszellstoffs nach alkalischer Extraktion an. Um den Einfluß der kombinierten Behandlung auf die Zellulose zu untersuchen wurden die Viskosität der Zellstoffproben untersucht. Die Viskositätsbestimmung erfolgt entsprechend den Vorschriften nach TAPPI. Die Angabe der Viskosität erfolgt in ml/g.

**Tabelle 12**

| **Versuchsauswertung** | | | |
|---|---|---|---|
| **Einfluß der Zellulasedosis auf Delignifizierung und Viskosität** | | | |
| **von Zellstoff (SW; K 16,6) bei Verwendung einer zellulasehaltigen Sequenz (CL)E.** | | | |

| Zellulasedosis U/g Zellstoff | Kappa | Delignifizierung (%) | Viskosität (ml/g) |
|---|---|---|---|
| 0 | 12,7 | 23,5 | 947 |
| 0,1 | 12,5 | 24,7 | 972 |
| 0,3 | 12 | 27,7 | 982 |
| 1 | 11,9 | 28,3 | 957 |
| 3 | 11,8 | 28,9 | 963 |
| 9 | 11,6 | 30,1 | 905 |
| 10 | 11,5 | 30,7 | 891 |
| 30 | 11,1 | 33,1 | 822 |
| 90 | 11 | 33,7 | 734 |

Durch Erhöhung der Zellulasedosis läßt sich bis zu einem Bereich von 9 U/g Zellstoff eine Verbesserung der Delignifizierbarkeit erzielen. Bei weiterer Erhöhung der Dosis wird keine Verbesserung mehr erreicht. Die Viskosität des Zellstoffs bleibt bis zu einer Dosierung von ca. 3 U/g nahezu konstant. Bei weiterer Dosiserhöhung tritt ein deutlicher Verlust an Viskosität auf. Bei geeigneter Dosierung (3 U/g im Beispiel) läßt sich der positive Zellulaseeffekt für eine Verbesserung der Delignifizierung verwenden ohne daß bereits eine Schädigung der Faser (Viskositätsabfall) auftritt.

### Beispiel 12

### Einfluß des pH-Wertes auf die Delignifizierung mittels eines Laccase-Mediator Systems (LMS^{®}) welches als Additiv Zellulase enthält.

Um den Einfluß des pH-Werts zu demonstrieren wurde die Delignifizierung in Ansätzen bei verschiedenen pH-Werten verglichen. In einem 500 ml Laborautoklaven wurde nachfolgend tabellarisch beschriebener Ansatz 2 Stunden lang bei 45°C (Wasserbad) unter Anlegen eines Sauerstoffpartialdrucks von 10 bar inkubiert ( CL-Stufe).

### (CL)-Stufe

### 5 g Softwood-Zellstoff (atro)

Stoffdichte: 10 %
Laccase: 15 IU/ g Zellstoff
Zellulase aus Trichoderma viride (Sigma): 2,5 U / g Zellstoff
Mediator: N-OH-Acetanilid: 10 mg / g Zellstoff
pH- Werte: 4, 5, 6, 7, 8
Gesamtflüssigkeitsvolumen: 50 ml

Nach der Inkubation wurden die Ansätze 60 min lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte der in diesem System delignifizierten Zellstoffe wurde der Grad der Delignifizierung bestimmt.

**Tabelle 13**

| | | |
|---|---|---|
| pH-Abhängigkeit der Delignifizierung mit einem Laccase Mediator System, welches als Additiv Zellulase enthält (Zellstoff hat nach E-Stufe Kappa 16,6) | | |

| pH-Wert | Kappa | Delignifizierung (rel %) |
|---|---|---|
| 4 | 12,9 | 22,3 |
| 5 | 12,5 | 24,7 |
| 6 | 11,1 | 33,1 |
| 7 | 14,7 | 11,4 |
| 8 | 16,3 | 1,8 |

Die Tabelle 13 zeigt, daß das Laccase-Mediator System, welches als Zusatz Zellulase enthält, ein deutliches pH-Profil mit einem Optimum für die gewählten Komponenten im Bereich von pH 6 in der Delignifizierung zeigt.

### Beispiel 13

### Einfluß des Mediators auf die Delignifizierung mittels eines Laccase-Mediator Systems (LMS^{®}) welches als Additiv Zellulase enthält.

In 500 ml Laborautoklaven wurden nachfolgend tabellarisch beschriebene Referenzansätze 2 Stunden lang bei 45°C (Wasserbad) unter Anlegen eines Sauerstoffpartialdrucks von 10 bar inkubiert ( L-Stufe).

### L-Stufe (Vergleichsversuch)

### 5 g Softwood-Zellstoff (atro)

Stoffdichte: 10 %
Laccase: 15 IU/ g Zellstoff
Mediator: N-OH-Acetanilid: 10 mg / g Zellstoff
pH- Wert: 6,0
Gesamtflüssigkeitsvolumen: 50 ml

Um den positiven Effekt eines enzymatischen Additivs, wie z.B. der Zellulase, zu bestimmen, wurde einem der L-Stufe entsprechenden Ansatz als weiterer Zusatz Zellulase von Trichoderma viride (Sigma) zugegeben (CL-Stufe), so daß der Ansatz wie folgt zusammengesetzt war:

### (CL)-Stufe

### 5 g Softwood-Zellstoff (atro)

Stoffdichte: 10 %
Laccase: 15 IU/ g Zellstoff
Zellulase: 2,5 U / g Zellstoff
Mediator: N-OH-Acetanilid: 10 mg / g Zellstoff
pH- Wert: 6,0
Gesamtflüssigkeitsvolumen: 50 ml

Beide Ansätze wurden anschließend 60 min lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte wurde der Grad der Delignifizierung bestimmt. Der Ausgangszellstoffs hatte einen Kappawert von 16,6 nach alkalischer Extraktion.

**Tabelle 14**

| | | | | |
|---|---|---|---|---|
| Delignifizierung (rel %) mit dem Laccase Mediator System, welches als Additiv Zellulase enthält im Vergleich zu einem zellulasefreien System | | | | |

| | NHA | HBT | Vio | HPI |
|---|---|---|---|---|
| ohne Zellulase LE | 23,3 | 9,1 | 15,2 | 6,1 |
| mit Zellulase (CL)E | 33,1 | 13,3 | 21,1 | 14,5 |

Der Zusatz von Zellullase steigert die Delignifizierung eines Softwood Zellstoffs gegenüber einer Zellulase freien Referenz in Gegenwart aller verwendeter Mediatoren.

### Beispiel 14

### Delignifizierung verschiedener Zellstoffe mit einem Laccase-Mediator System (LMS^{®}) welches als Additiv Zellulase und Xylanase enthält.

In 500 ml Laborautoklaven wurden nachfolgend tabellarisch beschriebene Referenzansätze 2 Stunden lang bei 45°C (Wasserbad) unter Anlegen eines Sauerstoffpartialdrucks von 10 bar inkubiert ( L-Stufe).

### L-Stufe (Vergleichsversuch)

### 5 g verschiedene Zellstoffe (atro)

Stoffdichte: 10 %
Laccase: 15 IU/ g Zellstoff
Mediator: N-OH-Acetanilid: 10 mg / g Zellstoff
pH- Wert: 6,0
Gesamtflüssigkeitsvolumen: 50 ml

Um den positiven Effekt mehrer gleichzeitiger enzymatischer Additiven , wie z.B. der Zellulase und der Xylanase , zu bestimmen, wurde der L-Stufe entsprechenden Ansätzen als weiterer Zusatz Zellulase von Trichoderma viride (Sigma) und Xylanase zugegeben (LXC-Sufe), so daß die Ansätze wie folgt zusammengesetzt waren:

### (LXC)-Stufe

### 5 g verschiedene Zellstoffe (atro)

Stoffdichte: 10 %
Laccase: 15 IU/ g Zellstoff
Zellulase: 0,1 U / g Zellstoff
Xylanase: 2,0 U/g
Mediator: N-OH-Acetanilid: 10 mg / g Zellstoff
pH- Wert: 6,0
Gesamtflüssigkeitsvolumen: 50 ml

Alle Ansätze wurden nach der Inkubation 60 min lang alkalisch extrahiert (E-Stufe)

### E-Stufe

### 2% Stoffdichte

Temperatur: 60°C
Alkali: 80g NaOH/ kg Zellstoff

Aus der Bestimmung der Kappa-Werte wurde der Grad der Delignifizierung bestimmt. Verglichen wurden die erreichbaren Delignifizierungsgrade (rel. %) einer Behandlung mit Laccase / Mediator (LE) (Vergleichsversuch) mit der Behandlung mit Laccase / Mediator / Zellulase / Xylanase (LXC)E.

**Tabelle 15**

| | | | | | |
|---|---|---|---|---|---|
| Vergleich von Delignifizierungen eines mit Zellulase und Xylanase Zusatz versehenen Laccase Mediator Systems - Sequenz (LXC)E - mit dem zusatzfreien System - Sequenz LE - . Getestet wurden verschiedene Zellstoffe. | | | | | |

| Zellstofftyp* | Kappa LE | Kappa (LXC) E | Delignif. LE | Delignif. (LXC) E | Delig. -Zun ahme (%) |
|---|---|---|---|---|---|
| SwKPu | 17,4 | 15,9 | 15,5 | 22,8 | 7,3 |
| SwKPuO | 10,5 | 9,3 | 30 | 38,3 | 8,3 |
| SwKPuO | 10,2 | 9,1 | 23,3 | 31,6 | 8,3 |
| SwKPuO | 6,2 | 5,1 | 31,9 | 44 | 12,1 |
| SwKPuO | 12,7 | 11,5 | 23,5 | 30,7 | 7,2 |
| SwKPuO | 9 | 8,1 | 28 | 35,2 | 7,2 |
| SwKPuO | 10,9 | 10,2 | 33,5 | 37,8 | 4,3 |
| SwKPu2O | 7,1 | 6,1 | 23,7 | 33,9 | 10,2 |
| HwKPu | 10,5 | 8,9 | 12,5 | 25,8 | 13,3 |
| HwKPuO | 7,6 | 7,1 | 29,6 | 34,3 | 4,7 |

| | | | | | |
|---|---|---|---|---|---|
| * SW Softwood HW Hardwood K Kraft O Sauerstoff behandelt Pu Pulp (Zellstoff) | | | | | |

Bei allen Zellstoffen kann eine deutliche Steigerung der Delignifizierung mittels Laccase Mediator System durch den gleichzeitigen Zusatz von Zellulase und Xylanase erzielt werden.

## Patentansprüche

1. Mehrkomponentensystem zum Verändern, Abbau oder Bleichen von Lignin, ligninhaltigen Materialien oder ähnlichen Stoffen, enthaltend eine Oxidoreduktase und ein für die Oxidoreduktase geeignetes Oxidationsmittel und einen Mediator und mindestens ein enzymatisch wirksames Additiv, dadurch gekennzeichnet, daß der Mediator die Oxidoreduktase und das enzymatisch wirksame Additiv nicht inaktiviert und das enzymatisch wirksame Additiv aus der Gruppe der Hydrolasen der Enzymklasse 3.2.1. ausgewählt ist.

2. Mehrkomponentensystem nach Anspruch 1, dadurch gekennzeichnet, daß der Mediator aus der Gruppe der aliphatischen, cycloaliphatischen, heterocyclischen oder aromatischen Verbindungen ausgewählt ist, die mindestens eine N-Hydroxy-, Oxim-, Nitroso-, N-Oxyl- oder N-Oxi-Funktion enthalten, wobei substituierte oder nichtsubstituierte 1-Hydroxy-1-benzotriazole, 3H-Benzotriazol-1-oxide und 2H-Benzotriazol-1-oxide ausgenommen sind.

3. Mehrkomponentensystem nach Anspruch 1, dadurch gekennzeichnet, daß der Mediator aus der Gruppe der Verbindungen gemäß Formeln I bis XXXIV der Beschreibung ausgewählt ist.

4. Mehrkomponentensystem nach Anspruch 1 bis 3, dadurch gekennzeichnet, daß das enzymatisch wirksame Additiv aus der Gruppe der Xylanasen, Mannanasen und Zellulasen ausgewählt ist.

5. Mehrkomponentensystem nach Anspruch 4, dadurch gekennzeichnet, daß es sich bei dem enzymatisch wirksamen Additiv um eine Endo 1,4-β-Xylanase (Enzymklasse 3.2.1.8) und/oder Endo 1,4-β-Glucanase (Enzymklasse 3.2.1.4) handelt.

6. Mehrkomponentensystem nach einem oder mehreren der Ansprüche 1 bis 5, dadurch gekennzeichnet, daß als Oxidoreduktase eine Laccase eingesetzt wird.

7. Verfahren zur Delignifizierung von ligninhaltigen Materialien, die dadurch gekennnzeichnet sind, daß mindestens eine Oxidoreduktase und mindestens ein für die Oxidoreduktase geeignetes Oxidationsmittel und mindestens ein Mediator der ein Enzym, ausgewählt aus der Gruppe der Oxidoreduktasen und Hydrolasen der Enzymklasse 3.2.1., nicht inaktiviert und mindestens eine Endohydrolase gleichzeitig oder in beliebiger Reihenfolge mit einer wässrigen Suspension des ligninhaltigen Materials mischt.

8. Verfahren gemäß Anspruch 7, dadurch gekennzeichnet, daß 0,1-100 IU Oxidoreduktase pro g ligninhaltiges Material (Trockengewicht) eingesetzt werden.

9. Verfahren gemäß Anspruch 7 oder 8, dadurch gekennzeichnet, daß der Mediator in Mengen von 1 - 500 mmol/kg Zellstoff eingesetzt wird.

10. Verfahren gemäß Anspruch 7 , dadurch gekennzeichnet , daß die Hydrolase der Enzymklasse 3.2.1. in Dosierungen von 0,01 - 1000 IU Enzym/g Zellstoff eingesetzt wird.
